(19) Europäisches Patentamt
European Patent Office
Office européen des brevets

(11) Publication number: **0 186 379 B1**

# EUROPEAN PATENT SPECIFICATION

(45) Date of publication of patent specification: **13.03.91**

(51) Int. Cl.⁵: **C12N 15/12, C07H 21/04, C12P 21/02, C07K 13/00, C12N 1/20, A01N 63/00, //(C12R1/07,1:19)**

(21) Application number: **85308994.4**

(22) Date of filing: **11.12.85**

(54) Gene for insecticidal protein.

(30) Priority: **12.12.84 JP 260638/84**

(43) Date of publication of application:
**02.07.86 Bulletin 86/27**

(45) Publication of the grant of the patent:
**13.03.91 Bulletin 91/11**

(84) Designated Contracting States:
**AT BE CH DE FR GB IT LI LU NL SE**

(56) References cited:
EP-A- 0 063 949
EP-A- 0 093 062

THE JOURNAL OF BIOLOGICAL CHEMISTRY,
vol. 258, no. 1, January 10, 1983
(Baltimore.USA) H.C.WONG et al.:
"Transcriptional and Translational Start
Sites forthe Bacillus thuringiensis Crystal
Protein Gene" pages 1960-1967

SCIENCE, vol. 219, no. 4585, February 11,
1983 (Washington DC) L.K.MILLER et al.:
"Bacterial, Viral and Fungal Insecti-

cides"pages 715-721

(73) Proprietor: **Suntory Limited**
**1-40 Dojimahama 2-chome Kita-ku**
**Osaka-shi Osaka-fu 530(JP)**

(72) Inventor: **Shibano, Yuji c/o SUNTORY LIMITED**
**Ohyobiseibutsu Kenkyusho 1-1**
**Wakayamadai 1-chome**
**Shimamoto-cho Mishima-gun Osaka(JP)**
Inventor: **Amachi, Teruo c/o SUNTORY LIMIT-**
**ED**
**Ohyobiseibutsu Kenkyusho 1-1**
**Wakayamadai 1-chome**
**Shimamoto-cho Mishima-gun Osaka(JP)**
Inventor: **Yoshizumi, Hajime c/o Suntory Ltd.**
**Ohyobiseibutsu Kenkyusho 1-1**
**Wakayamadai 1-chome**
**Shimamoto-cho Mishima-gun Osaka(JP)**

(74) Representative: **Ford, Michael Frederick et al**
**MEWBURN ELLIS & CO. 2/3 Cursitor Street**
**London EC4A 1BQ(GB)**

**Description**

BACKGROUND OF THE INVENTION

1. Field of the Invention

The present invention relates to genes coding for insecticidal proteins, plasmids containing the genes, and microorganisms transformed with the plasmids.

2. Description of the Related Art

Since an insecticidal protein produced by Bacillus thuringiensis shows toxicity against larvae of Lepidoptera, preparations containing the insecticidal protein have been commercially provided as insecticides with selective toxicity to insects. As the insecticidal protein, however, is produced only during the sporulation period, by Bacillus thuringiensis, the production period is rather short, and this limits the productivity of the protein. Therefore, the microbial insecticide is higher in cost than conventional chemically synthesized insecticides.

Moreover, the insecticidal protein produced by Bacillus thuringiensis is characteristically crystalline, and has a poor solubility in water. Accordingly, it is difficult to economically separate and purify the protein from the spores of producer cells. Therefore, the conventional microbial insecticides are prepared as formulations containing spores of Bacillus thuringiensis, which spores will pollute the environment when the formulation is used.

To overcome the drawbacks of the conventional microbial insecticides, approaches involving gene recombination techniques have been proposed. Namely, Wong et. al. J. Biol. Chem. 258, 1960 (1983) describes a part of a nucleotide sequence of an amino-terminal of insecticidal protein of Bacillus thuringiensis subsp. Kurstaki HD-I-Dipel origin. However, the nucleotide sequence is different from that of the present invention at three sites, which provides the difference of three amino acid residues in the protein. JP-A-58-500565 (equivalent to EP-A-63949 and WO 82/03872), refers to a DNA fragment coding for an insecticidal protein of Bacillus thuringiensis subsp. sotto origin, but does not include a detailed description thereof. Moreover, the molecular weights of a plasmid included in the Bacillus thuringiensis subsp. sotto, and of the produced insecticidal protein, are different from those of the present invention.

SUMMARY OF THE INVENTION

Therefore, as a means for production of a protein which has a insecticidal activity equivalent to that produced by Bacillus thuringiensis, is soluble in water, and can be produced over a long period when culturing a producer microorganism, it is sought to provide gene systems coding for the above-mentioned protein.

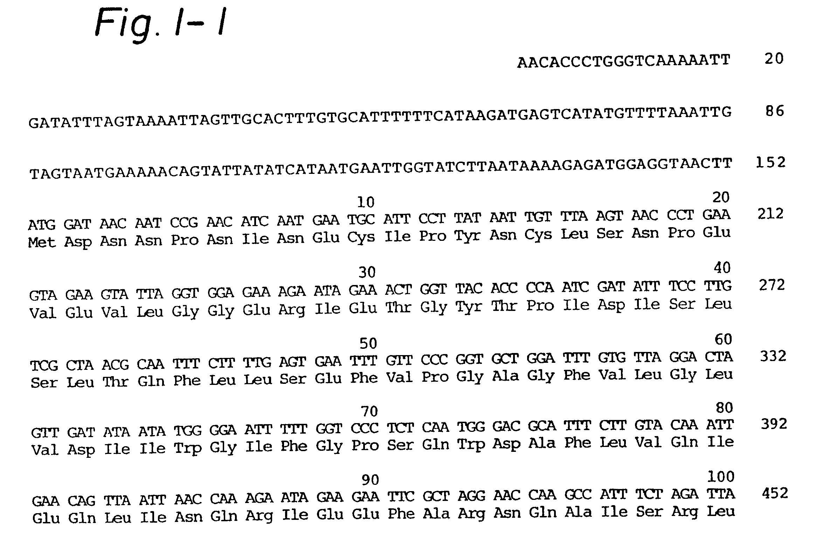

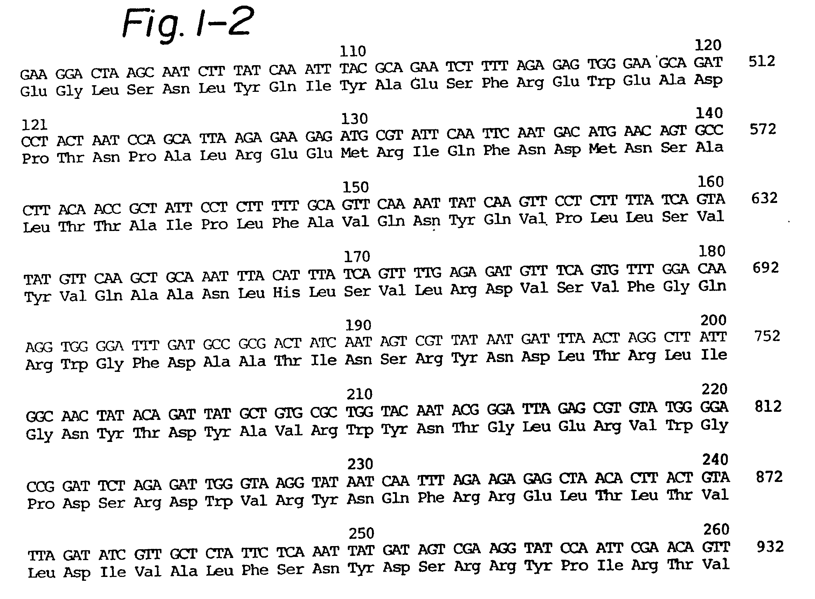

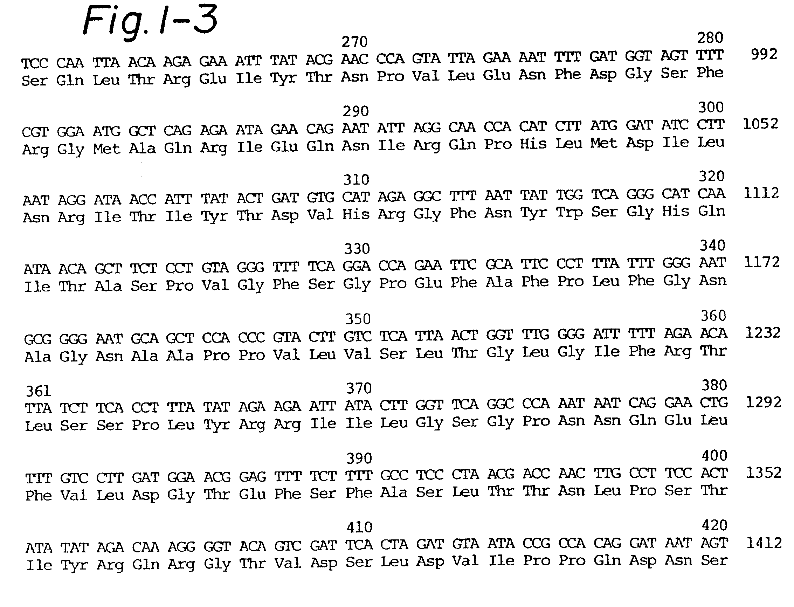

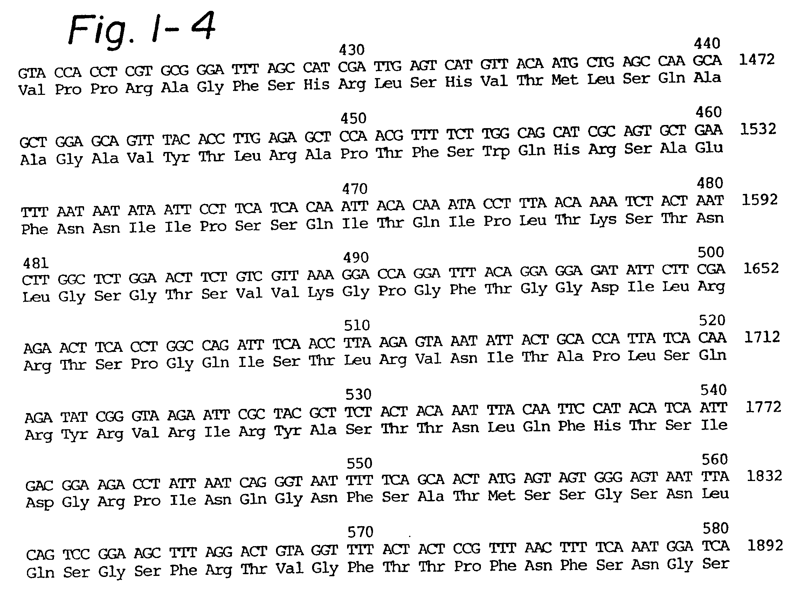

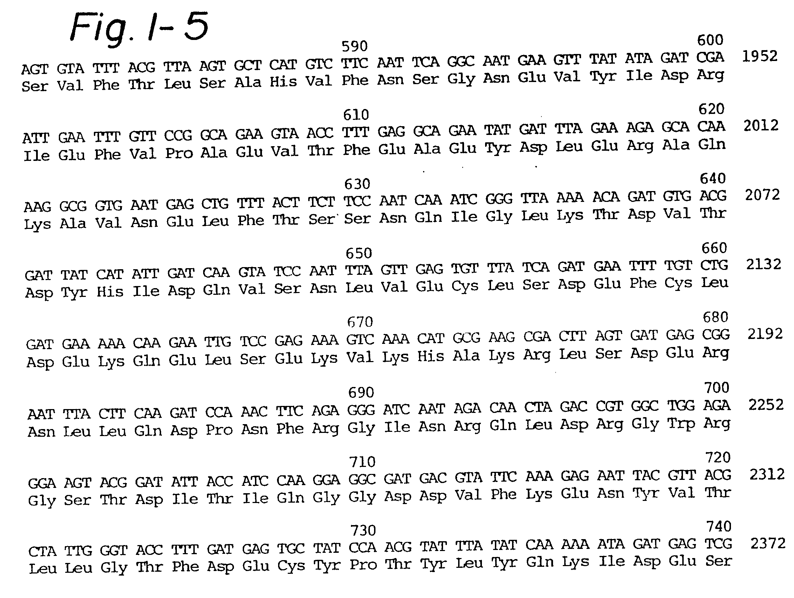

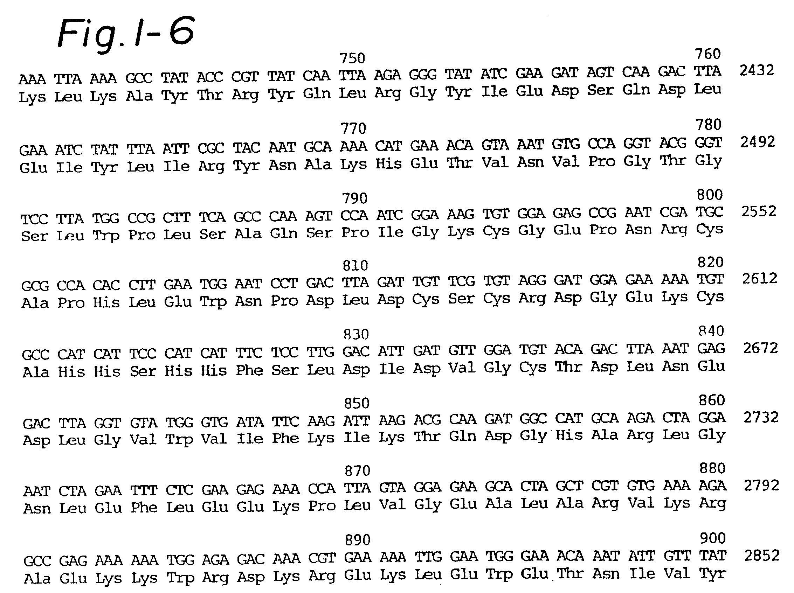

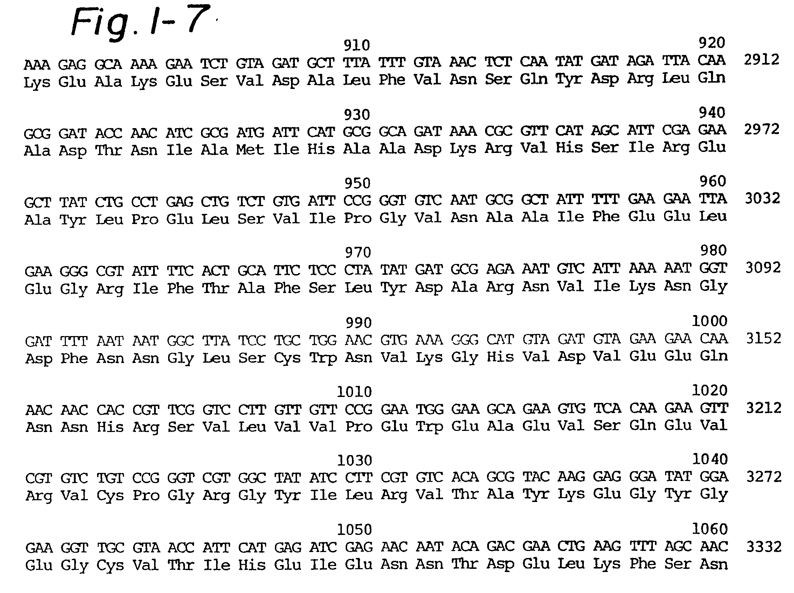

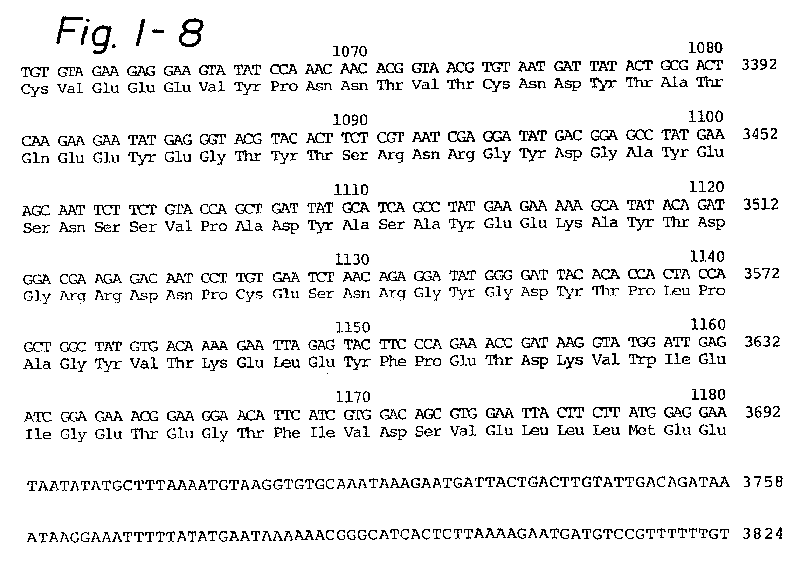

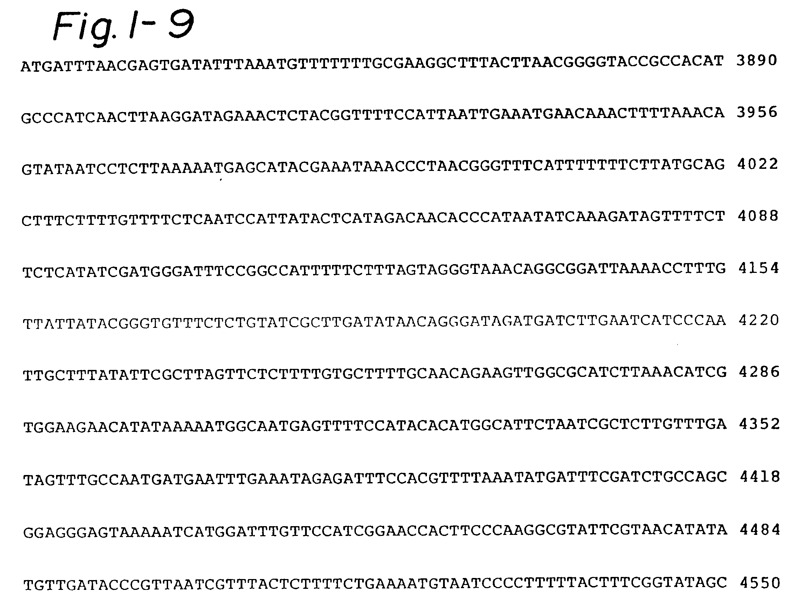

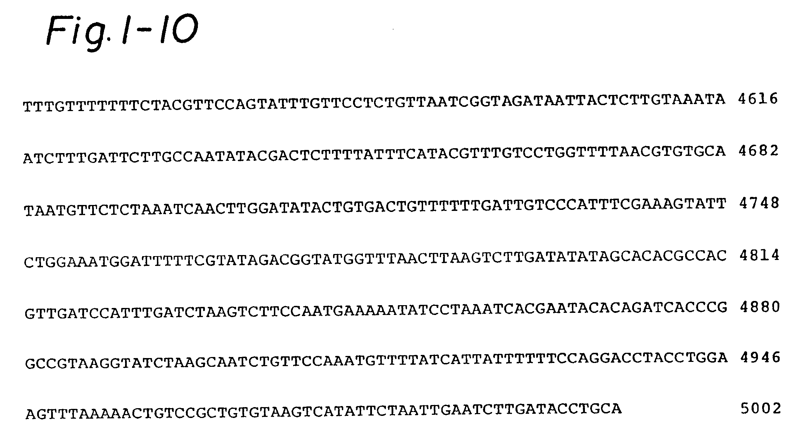

In one aspect the invention provides a DNA fragment comprising a nucleotide sequence coding for an insecticidal protein having an amino acid sequence as set out in Fig. 1, or having an amino acid sequence comprising at least 723 amino acids from the N-terminus thereof.

In a second aspect the invention provides a DNA fragment having the nucleotide sequence from position 153 to position 3692 (codons 1 to 1180) as set out in Fig. 1, or at least 723 codons from the 5'-terminus of that sequence.

In a third aspect the invention provides a plasmid capable of replicating in E. coli and expressing the insecticidal protein defined above, comprising a pBR322 plasmid in which a DNA sequence as defined has been inserted.

In a fourth aspect the invention provides E. coli transformed with the plasmid so as to produce insecticidal protein.

In a fifth aspect the invention provides water-soluble insecticidal protein produced by an organism transformed with said DNA.

In further aspects the invention provides processes comprising the production of the DNA fragments, plasmids and proteins described.

## BRIEF DESCRIPTION OF THE DRAWINGS

Figures 1-1 to 1-10 represent a nucleotide sequence of a 5 kilo base (kb) insert of DNA fragment in plasmid pSP801 including an open reading frame, and an amino acid sequence deduced from a nucleotide sequence corresponding to the open reading frame.

Figure 2 represents a restriction map of a starting plasmid pSY1 derived from Bacillus thuringiensis subsp. sotto.

Figure 3 represents a flow chart of the construction of plasmids according to the present invention.

Figure 4 represents a restriction map of plasmid pSP801.

Figure 5 schematically represents a principle for determination of a region coding for an insecticidal protein in 6.6 kb insert of DNA fragment in the plasmid pSP801.

Figure 6 represents a restriction map of 2.95 kb insert of DNA fragment in plasmid pLBT291.

Figure 7 is a photograph of electrophoresis showing expression of insecticidal protein by plasmid pLBT291 (lane 1) , pKB5 (lane 2), pKA2 (lane 3), pMR4 (lane 4), pHP1 (lane 5), pSP801 (lane 6), pSX8 (lane 7), and pBR322 (lane 8, control).

## DESCRIPTION OF THE PREFERRED EMBODIMENT

The DNA fragment coding for an insecticidal protein according to the present invention is derived from a plasmid pSY1 present in Bacillus thuringiensis subsp. sotto and including a gene for insecticidal protein.

The plasmid pSY1 is isolated from the above-mentioned strain according to a modification of the method described by Hansen and Olsen, J. Bacteriol. 135, 227-238 (1978) . The molecular size of the plasmid is calculated to be 68 kb.

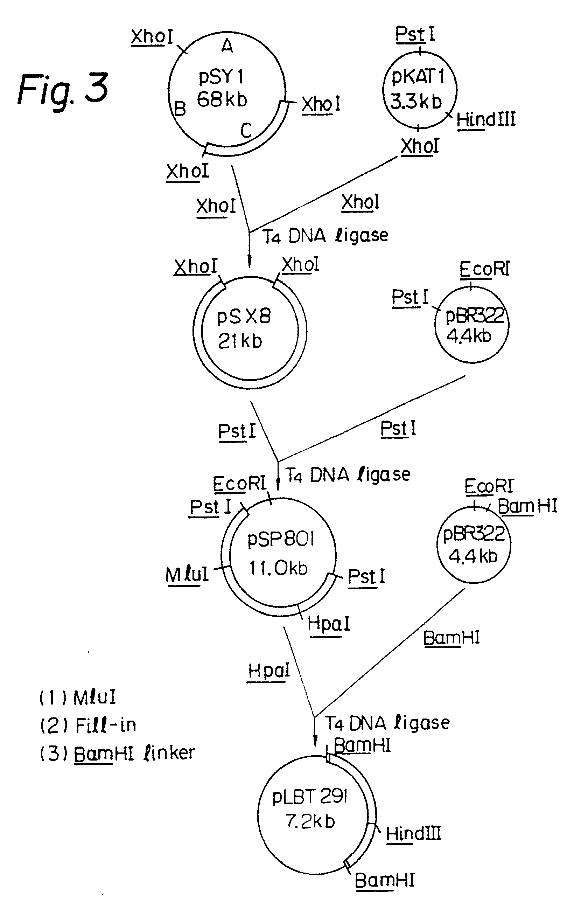

As shown in Fig. 3, the plasmid pSY1 is cleaved with a restriction enzyme XhoI to obtain a DNA fragment containing a gene coding for insecticidal protein. The DNA fragment is then inserted into an XhoI site of a cloning vector pKAT 1 The ligation mixture is used to transform E. coli C600, and the transformants are screened for ampicillin resistance and the production of insecticidal proteins as determined by radioimmunoassay to obtain a clone containing plasmid pSX8, which includes an 18 kb insert containing a nucleotide sequence coding for the insecticidal protein.

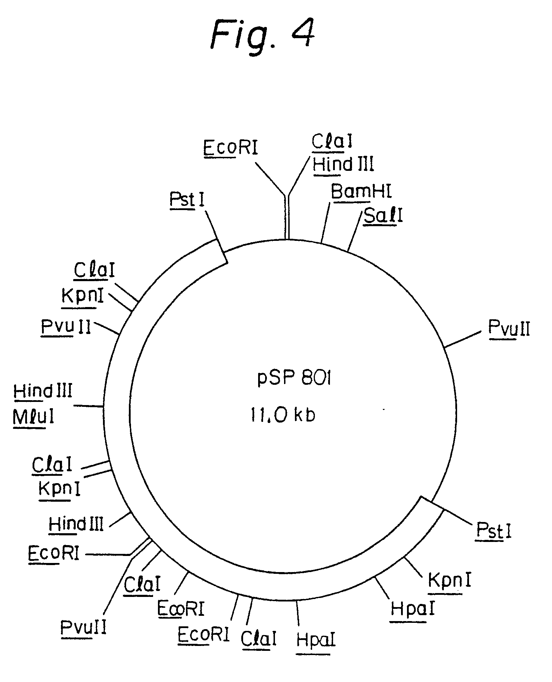

The plasmid pSX8 is cleaved with various kinds of restriction enzymes, and each of the DNA fragments thus obtained is ligated to a cloning vector pBR322 cleaved with the corresponding restriction enzyme. Each of the ligation mixtures is used to transform E. coli HB101, and the transformants are screened as described above to obtain a clone E. coli HB101/pSP801, which contains a plasmid pSP801 of about 11.0 kb including a 6.6 kb PstI DNA insert. A restriction map of the plasmid pSP801 is represented in Fig. 4.

As E. coli HB101/pSP801 produces a polypeptide having a molecular weight of about 144 kilo Daltons (kD), and this molecular weight is approximately the same as that of a crystal protein produced by Bacillus thuringiensis, the insert of about 6.6 kb in the plasmid pSP801 is supposed to contain a nucleotide sequence coding for the entire amino acid sequence of a protein, the same as the crystal protein produced by Bacillus thuringiensis.

The plasmid pSP801 is then cleaved with restriction enzymes M1uI and HpaI to obtain a DNA fragment of about 2.95 kb, which is then filled-in, and added with Bam HI linker at both terminals. The DNA fragment thus modified is then inserted into a Bam HI site of pBR322 to obtain a ligation mixture. The mixture is used to transform E. coli HB101, and the transformants are screened as described above to obtain a clone E. coli HB101/pLBT291.

The clone E. coli HB101/pLBT291 produces a polypeptide of about 75 kD having insecticidal activity. Therefore, the above-mentioned DNA fragment of about 2.95 kb is confirmed to contain a nucleotide sequence coding for a part of the amino acid sequence of the insecticidal crystal protein produced by Bacillus thuringiensis.

A nucleotide sequence of the DNA fragment of about 2.95 kb corresponds to a nucleotide sequence at positions 1 to 2955 in Fig. 1-1 to 1-7. The DNA fragment consists of 2955 bases, in which 2802 bases from the positions 153 to 2954 encode 934 amino acids corresponding to the above-mentioned polypeptide. Another part of the DNA fragment consisting of nucleotides from the positions 1 to 152 is a 5' non-coding sequence. The nucleotide sequence is different from the nucleotide sequence disclosed by Wong et. al., supra , at three sites, which difference results in a difference of three amino acids in the peptide.

Examples

The present invention will now be further illustrated by, but is no means limited to, the following examples.

### (1) Preparation of plasmid pSY1

Plasmid pSY1 was prepared from Bacillus thuringiensis subsp. sotto according to a modification of the alkali-method described by Hansen and Olsen, J. Bacteriol. 135, 227-238 (1978). The above-mentioned bacterium was cultured in 400 ml of Penassay broth, and the cultured cells were suspended in 10.8 ml of a solution comprised of 25% sucrose, 50 mM Tris-HCl, and 20 mM EDTA (pH 8.0), and the suspension was added with 10 mg/ml of lisozyme and incubated at 37° C for one hour. The suspension was then added with pronase E (T.M) and sodium dodecyl sulfate (SDS) to concentrations of 0.5 mg/ml and 1% respectively, and incubated at the same temperature for another one hour to lyse the cells completely. The lysate was added with 3 ml of 3N NaOH and incubated at a room temperature for three minutes, and then added with 8 ml of 2M Tris-HCl (pH 7.0), 4 ml of 20% SDS, 8 ml of 5M NaCl, and incubated for six hours in ice. The thus-treated lysate was centrifuged at 17,000 x G for 30 minutes to obtain a supernatant. To the supernatant, polyethylene glycol 6000 was added to a concentration of 10% to precipitate DNA.

DNA was dissolved in 20 ml of a solution comprised of 20 mM Tris-HCl, 5 mM EDTA and 150 mM NaCl (ph 7.5), and the solution was added with RNase A to a concentration of 0.1 mg/ml, incubated at 37° C for two hours, extracted with phenol, and added with two volumes of ethanol to precipitate DNA. The precipitate was dissolved in 5 ml of a solution comprising 20 mM Tris-HCl, 5 mM EDTA, and 150 mM NaCl (pH 7.5), and the solution was passed through a Biogel A-0.5 m (T.M.) column, and a void volume fraction was recovered. To this fraction, ethanol was added to precipitate DNA, which was then purified by density-equilibrium centrifugation in ethidium bromide-cesium chloride. The plasmid DNA thus obtained showed a single band under agarose gel electrophoresis.

### (2) Restriction map of plasmid pSY1

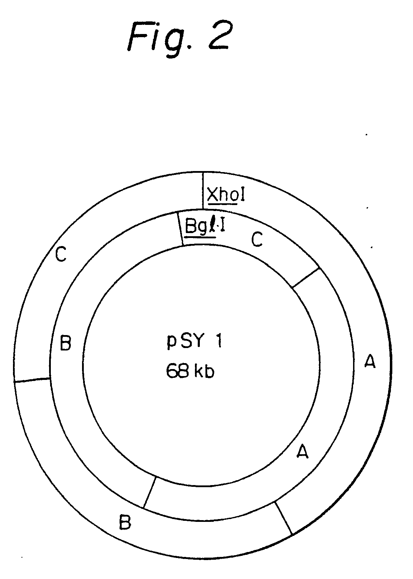

DNA of plasmid pSY1 was treated with restriction enzymes. The DNA was cleaved with Bam HI and Stu I at one site, with Bgl II and Xho I at three sites, and with Mlu I and Sac I at four sites, but not cleaved with Aat II, Sal I and Sma I. Bon I, Bcl II, Cla I, Eco RI, Hind III, Hpa I, Kpn I, Pst I and Pvu II provided many fragments. Figure 2 represents a restriction map obtained by Bgl II and Xho I. Cleavage sites of Bam HI and Stu I locate on a Bgl II-A fragment and an Xho I-A fragment, respectively, at a distance of about 2 kb. On the basis of the size of DNA fragments, the plasmid pSYl was found to be 68 kb in size.

### (3) Selection of Transformant by Radioimmunoassay

In the plasmid construction described below, E. coli transformed with a plasmid containing a gene for an insecticidal protein were selected according to a modification of the procedure described by Erlich et. al., Cell, 13 , 681-689 (1978) using an antibody against the insecticidal protein.

Bacillus thuringiensis subsp. sotto was cultured in a modified G medium described by Aronson et. al., J. Bacteriol. 106, 1016-1025 (1971) for three days, and the insecticidal crystal protein was isolated and purified from the culture according to a method of Sharpe et. al., Appl. Microbiol. 30, 1052-1053 (1975). The purified protein was used as an antigen to prepare the antibody. The antibody IgG thus obtained was labeled with 125I according to a procedure described by Marrison and Bayse, Biochemistry 9 , 2995-3000 (1970).

Cells of E. coli to be tested were cultured on an agar plate medium to form colonies, and the agar plate carrying the colonies thereon was exposed to vapor of chloroform for 30 minutes, and overlaid with an agar layer containing 1 mg/ml lysozyme, 10 mM EDTA and 0.02% Triton X100 (T.M) to lyse cells of the colonies. On the overlaid agar plate was placed a DBM filter paper on which the unlabeled antibody had been bonded according to a procedure described by Alwine et. al., Proc. Natl. Acad. Sci. USA, 74, 5350-5354 (1977), and allowed to stand for 12 to 15 hours. The filter paper was washed with 10 mM phosphate buffer pH 7.2 containing 5% bovine serum and 0.15M NaCl (CS-PBS), and maintained in 10 mN Tris-HCl buffer pH 7.5 containing 0.14M NaCl, 25% bovine serum and 4 x 10^6 cpm 125I-labeled antibody prepared as described above for 5 to 7 hours. The filter was then washed with CS-PBS, dried, and exposed on an X-ray film to carry out autoradiography.

4

(4) Construction of plasmid pSX8

The route for the construction of plasmid pSX8 is shown in Fig. 3. The plasmid pSY1 was cleaved with a restriction enzyme XhoI to obtain DNA fragments. A cloning vector plasmid pKAT1 was cleaved with Xho I. The DNA fragments from pSY1 were then inserted to Xho I site of the cleaved pKAT1 with T4 DNA ligase to obtain a ligation mixture, which was then used to transform cells of E. coli C600. The transformants were first screened on L broth agar plate medium supplemented with 25 μg/ml ampicillin to select clones resistant to ampicillin, the selected clones were then subjected to the radioimmunoassay described in paragraph (3), and a positive clone containing recombinant plasmid pSX8 was selected. The plasmid pSX8 contains a 18 kb insert of DNA fragment, which includes a gene coding for an insecticidal protein.

(5) Construction of plasmid pSP801

The plasmid pSX8 was cleaved with a restriction enzyme Hind III, Pst I, or Eco RI to obtain DNA fragments, which were then inserted into a corresponding restriction site of cloning vectors pBR322 and pBR325 cleaved with the corresponding restriction enzyme, and each ligation mixture was used to transform E. coli HB101. The transformants thus obtained were then screened according to the above-described procedures to select a clone expressing an insecticidal protein. As a result, from the ligation of Pst I - cleaved pSX8 DNA fragment with Pst I - cleaved pBR322 fragment, a clone E. coli HB101/pSP801 containing plasmid pSP801 was selected. The route for construction of the plasmid pSP801 is shown in Fig. 3, and a restriction map of the plasmid pSP801 is shown in Fig. 4. The plasmid pSP801 contains an inserted Pst I DNA fragment of 6.6 kb, which includes a gene coding for an insecticidal protein.

(6) Construction of plasmid pLBT291

As shown in Fig. 3, plasmid pSP801 was cleaved with Mlu I and Hpa I, and the Mlu I - HPa I DNA fragment thus obtained was filled-in, and attached with Bam HI linkers at both terminals. The Bam HI fragment was then inserted into a Bam HI site of pBR322. The ligation mixture thus prepared was used to transform E. coli HB101. The transformants were then screened according to the radioimmunoassay described in paragraph (3) to obtain a clone E. coli HB101/pLBT291, containing plasmid pLBT291. It was confirmed that the clone produced an insecticidal protein, by the bioassay described below in detail in paragraph (9). The plasmid pLBT291 contained a 2.95 kb insert of DNA fragment which corresponds to the Hpa I•2 - Mlu I fragment on plasmid pSP801.

(7) Determination of location of gene coding for insecticidal protein

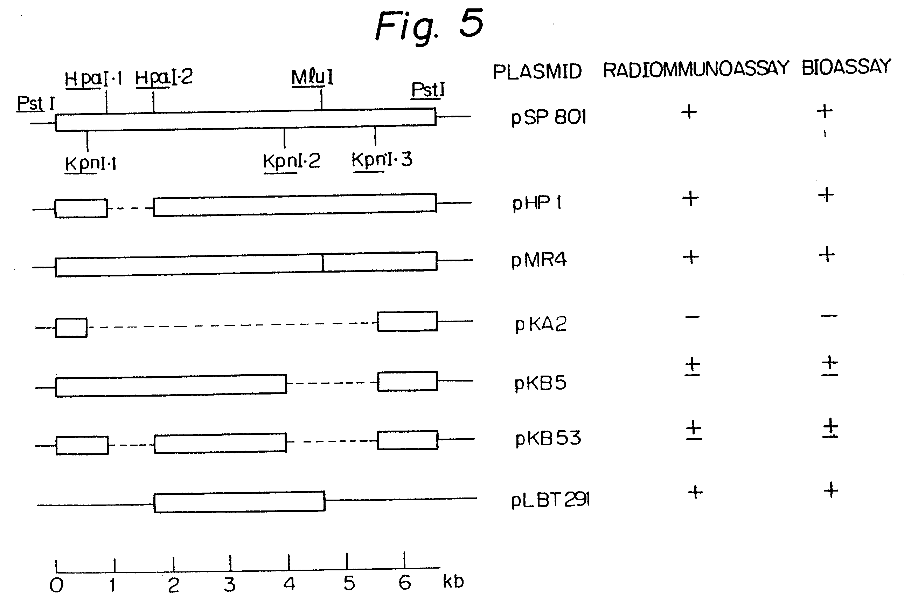

To determine a location of gene coding for an insecticidal protein on the 6.6-kb insert of pSP801, deletion derivatives were constructed from pSP801 using the restriction sites indicated in Fig. 5. Each of these derivatives was used to transform E. coli HB101, and a clone containing plasmid pHP1, pMR4, pKA2, pKB5, or pKB53 was obtained. Among these plasmids, as shown in Fig. 5, plasmid pMR4 contained all regions of the 6.6 kb-PstI DNA fragment and 4 base pairs insertion at the Mlu I site, but other plasmids contained only parts of the PstI DNA fragment.

Each of the clones was cultured, and tested for the production of an insecticidal protein by the radioimmunoassay described in paragraph (3) and bioassay described below in paragraph (9).

The results obtained from the above-mentioned tests are summarized in Fig. 5, together with the result obtained from pSP801 and pLBT291.

In Fig. 5, the left part of the drawing represents profiles of a region of each plasmid corresponding to the 6.6-kb Pst I DNA fragment in plasmid pSP801. The right side columns represent the results of the radioimmunoassay and bioassay respectively, wherein + denotes positive, - denotes negative, and + denotes intermediate activity. From, Fig. 5, it is confirmed that the gene coding for an aimed insecticidal protein is located on the 2.95 kb Hpa I•2 - Mlu I fragment in pSP801. This was confirmed by a direct cloning of the 2.95 Hpa I•2 - Mlu I fragment into the Bam HI site of pBR322 using a Bam HI linker. The resulting plasmid pLBT291 was positive in both the radioimmunoassay and bioassay.

(8) Electrophoretic analysis of gene products produced in E. coli cells

The clones which were pronounced positive in the radioimmunoassay, i.e., E. coli HB101/pSX8, E. coli HB101/pS801, E. coli HB101/pLBT291, and other clones containing pKB5, pKA2, pMR4 and pHP1 respectively, as well as the control clone, i.e., E. coli HB101/pBR322, were cultured in 400 ml of L-broth medium supplemented with 25 μg/ml of ampicillin, overnight at 37°C, and the cultured cells were collected by centrifugation and suspended in a buffer solution containing 150 mM NaCl, 20 mM Tris-HCl (pH8.0), 5 mM EDTA, 2 mM phenylmethylsulfonyl fluoride, and 7 mM mercaptoethanol. The suspension was added with 0.3 ml of 10 mg/ml lysozyme, maintained on ice for 30 minutes, and sonicated for three minutes to rupture the cells. The sonicated product was then centrifuged at 14,000 xg for 20 minutes to recover a supernatant. The supernatant was then dialysed overnight in 20 mM phosphate buffer pH7.2. The extracts thus obtained from the cultured E. coli were subjected to electrophoresis and bioassay. In the electrophoresis, the extracts were separated on 7% SDS-polyacrylamide gel according to a conventional procedure. The separated products were electrophoretically transferred to a nitrocellulose filter, and the nitrocellulose filter was incubated successively with an antibody against the insecticidal crystalline protein, anti-rabbit IgG antibody labeled with peroxidase, and then with a substrate solution for the peroxidase. In this manner, only polypeptides having reactivity with the antibody against the insecticidal protein bind the antibody, which in turn binds the anti-rabbit IgG labeled with peroxidase, which acts on its substrate to develop color showing the presence of the aimed polypeptide on the nitrocellulose filter.

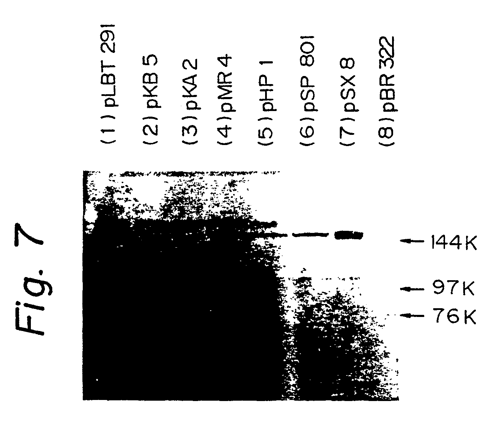

The electrophoresis patterns are shown in Fig. 7, wherein lane 1 represents a product from a clone containing plasmid pLBT291, lane 2 represents that from pKB5, lane 3 represents that from pKA2, lane 4 represents that from pMR4, lane 5 represents that from pHP1, lane 6 represents that from pSP801, lane 7 represents that from pSX8, and lane 8 represents that from pBR322. Although the insecticidal protein produced by Bacillus thuringiensis has been known to comprise three polypeptides, i.e., a main polypeptide having a molecular weight of 144 kD, and two minor polypeptides having a molecular weight of 97 kD and 76 kD respectively, the plasmids pMR4 (lane 4), pHP1 (lane 5), pSP801 (lane 6), and pSX8 (lane 7) provided a band corresponding to 144 kD, which corresponded to the main polypeptide of the insecticidal protein produced by Bacillus thuringiensis. On the other hand, plasmid pLBT291 provided a band corresponding to 75 kD. Plasmids pKB5 and pKA3, which were not pronounced positive in the radioimmunoassay and bioassay, and the control plasmid pBR322, provided no clear bands.

The above-mentioned result shows that the Pst I fragment in pSP801 and in pMR4, and the Hpa I.2 - Pst I fragment in pHP1 contain a DNA sequence coding for a full length polypeptide of 144 kD. On the other hand, since the Hpa I.2 - Mlu I fragment of 2.95 kb in pLBT291 corresponds to a 5'-terminal side part of the Hpa I.2 - Pst I fragment in pHP1, the Hpa I.2 - Mlu I 2.95 kb fragment in pLBT291 should encode a polypeptide corresponding to a part of the 144 kD polypeptide.

In this connection, it should be noted that both the 75 kD polypeptide derived from pLBT291 and the 144 kD polypeptide derived from other plasmids react with the antibody against the insecticidal protein obtained from Bacillus thurringiensis. This suggests that the 75 kD polypeptide contains an essential region or site for the insecticidal activity.

(9) Bioassay for insecticidal activity of gene product from transformed E. coli

The extracts prepared as described in paragraph (8) from E. coli HB101/pBR322, E. coli HB101/pSX8, E. coli HB101/pSP801, and E. coli HB101/pLBT291 were tested for their insecticidal activity.

1 ml of each extract was impregnated into 10 g of an artificial feed composition for silkworms, and the impregnated feed composition was put in a plastic container, into which five fifth instar silkworms were incorporated. The container was allowed to stand overnight, and the viability rate and an amount of increase of the body weight of the silkworms were determined. The results are set forth in the following table.

Table

| Extract derived from following plasmid ' | Number of viable silkworms per total silkworms | Increase of body weight (g) (2) |
|---|---|---|
| Control(1) | 5/5 | 2.86 |
| pBR322 | 5/5 | 2.45 |
| pSX8 | 0/5 | 0.38 |
| pSP801 | 0/5 | 0.53 |
| pLBT291 | 0/5 | 0.91 |

(1)   Control means that the feed composition was impregnated with 1 ml of 20 mM phosphate buffer solution.

(2)   The gram increase of body weight is a total of five silkworms.

As seen from the table, all of the silkworms which ate the feed impregnated with the extract prepared from E. coli transformed with pSX8, pSP801 or pLBT291 died, and a significant increase of weight was not observed. On the other hand, all of the silkworms which ate the feed impregnated with phosphate buffer solution or with the extract derived from plasmid pBR322 were viable and a substantial increase of body weight was observed.

It should be noted that the 75 kD polypeptide expressed by pLBT291 shows an insecticidal activity substantially identical to that of the 144 kD polypeptide which corresponds to the main polypeptide of the insecticidal crystal protein produced by Bacillus thuringiensis. This means that the 75 kD polypeptide contains a site or region essential for the insecticidal activity. Moreover, it should be noted that, in contrast with the natural insecticidal crystal protein produced by Bacillus thuringiensis, which is almost insoluble in water, both the 144 kD polypeptide and 75 kD polypeptide expressed by genes of the present invention are soluble in water.

(10) Determination of nucleotide sequence of DNA fragment containing gene coding for insecticidal protein

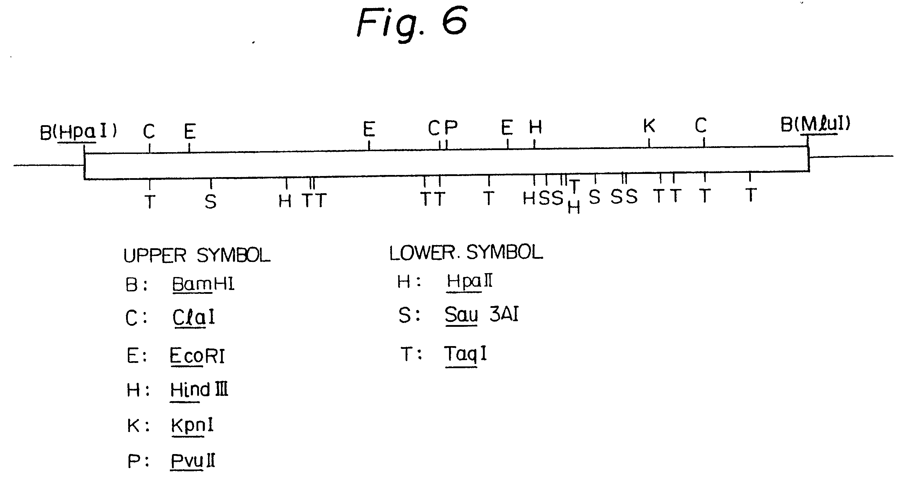

The sequence of the Hpa I.2 - Mlu I DNA fragment of 2.95 kb in plasmid pSP801, corresponding to the Bam HI DNA fragment of 2.95 bp in plasmid pLBT291 was determined according to the dideoxy method described by Messing, Method in Enzymology Vol. 101C, 20-78. A detailed restriction map of the Hpa I.2 - Mlu I fragment is set forth in Fig. 6.

The 2.95 kb DNA fragment was cleaved with restriction enzymes Sau 3A, Hpa II, and Taq I, and the resulting DNA fragments were cloned into phage M13mp10, or M13mp11. Moreover, the 2.95 kb DNA fragment was cleaved with restriction enzymes Cla I, Hind III, and Eco RI, and the resulting DNA fragments were cloned into phage M13mp10 or M13mp11. These cloned minute DNA fragments were sequenced according to the above-cited Messing's method, and the determined sequences were overlapped to determine a whole nucleotide sequence of the 2.95 kb DNA fragment. Moreover, the Mlu I - Pst I DNA fragment of 2 kb positioned adjacent to the 3'-terminal of the Hpa I.2 - Mlu I 2.95 kb fragment was sequenced as above-mentioned. In this manner, finally, the whole nucleotide sequence of the 5 kb DNA fragment from the Hpa I.2 site to the Pst I site was determined.

The nucleotide sequence thus determined is set forth in Fig. 1-1 to Fig. 1-10. The 5 kb DNA fragment consists of 5,002 bp, and contains a single large open reading frame starting from a starting codon ATG, wherein base "A" corresponds to the position 153 in the sequence in Fig. 1-1, and terminating at a stop codon TAA, wherein the last base "A" corresponds to the position 3,695 in the sequence in Fig. 1-8. The open reading frame consists of 3,540 bp, and is flanked by a 5'-non-translation sequence consisting of 152 bp, and by a 3'-flanking sequence consisting of 1,310 bp. There is no other open reading frame corresponding to the molecular weight of the insecticidal protein. Therefore the 144 kD insecticidal polypeptide should be derived from the above-mentioned open reading frame. The open reading frame consisting of 3,540 bp encodes 1180 amino acids. Therefore, the 144 kD insecticidal polypeptide is assumed to consist of 1180 amino acid residues starting from Met at the amino acid position 1 and terminating by Glu at the amino acid position 1180, which corresponds to 133,481 Dalton of the molecular weight. This amino acid sequence reveals that approximately half at the amino terminal side of the 144 kb insecticidal polypeptide contains much less lysine and cysteine than a carboxy terminal side does.

In the large open reading frame, a nucleotide sequence starting from base "A" at position 153 and terminated by "G" at the position 2954, consisting of 2802 bases, corresponds to an open reading frame contained in the 2.95 kb Hpa I.2 - Mlu I DNA fragment. The nucleotide sequence consisting of 2802 bases should encode 934 amino acids. Therefore, the polypeptide produced of E. coli transformed with plasmid pLBT291 may consist of at least 934 amino acid residues starting from Met at the amino acid position 1 to Arg at the amino acid position 934.

An inverted repeat sequence capable of forming a stable stem and loop structure is found from the position 3782, which corresponds to about 100 bp downstream from the stop codon, to the position 3824, and assumed to act as a terminater on transcription. Moreover, an open reading frame reversely extending from the position 5002 to the position 4021 is found.

According to the present invention, the insecticidal protein are provided in a water-soluble form. Therefore, the protein can be easily separated and purified from the producer cells. This means that insecticidal compositions specific to Lepidoptera can be produced in a form containing no producer microorganism's cells or spores. Such compositions, when used, are safe for the environment.

Moreover, since E. coli transformed with the present plasmid produces the insecticidal protein without the limitation in the production period, in contrast with Bacillus thuringiensis which produces it only within the spore forming period, according to the present invention, the insecticidal protein can be produced at a lower cost.

Finally, the nucleotide sequences and plasmids of the present invention can be further manipulated or modified to construct improved nucleotide sequences or plasmids which will provide new polypeptides having stronger insecticidal activity.

## Claims

1. A DNA fragment comprising a nucleotide sequence coding for an insecticidal peptide having the following amino acid sequence:

```
        1                                      10
Met Asp Asn Asn Pro Asn Ile Asn Glu Cys Ile Pro Tyr Asn Cys

                    20                                      30
Leu Ser Asn Pro Glu Val Glu Val Leu Gly Gly Glu Arg Ile Glu

                                    40
Thr Gly Tyr Thr Pro Ile Asp Ile Ser Leu Ser Leu Thr Gln Phe

                    50                                      60
Leu Leu Ser Glu Phe Val Pro Gly Ala Gly Phe Val Leu Gly Leu

                                    70
Val Asp Ile Ile Trp Gly Ile Phe Gly Pro Ser Gln Trp Asp Ala

                        80                                  90
Phe Leu Val Gln Ile Glu Gln Leu Ile Asn Gln Arg Ile Glu Glu

                                    100
Phe Ala Arg Asn Gln Ala Ile Ser Arg Leu Glu Gly Leu Ser Asn

                    110                                    120
Leu Tyr Gln Ile Tyr Ala Glu Ser Phe Arg Glu Trp Glu Ala Asp

                                    130
Pro Thr Asn Pro Ala Leu Arg Glu Glu Met Arg Ile Gln Phe Asn

                    140                                    150
Asp Met Asn Ser Ala Leu Thr Thr Ala Ile Pro Leu Phe Ala Val

                                    160
Gln Asn Tyr Gln Val Pro Leu Leu Ser Val Tyr Val Gln Ala Ala

                    170                                    180
Asn Leu His Leu Ser Val Leu Arg Asp Val Ser Val Phe Gly Gln

                                    190
Arg Trp Gly Phe Asp Ala Ala Thr Ile Asn Ser Arg Tyr Asn Asp

                    200                                    210
Leu Thr Arg Leu Ile Gly Asn Tyr Thr Asp Tyr Ala Val Arg Trp
```

Tyr Asn Thr Gly Leu Glu Arg Val Trp Gly Pro Asp Ser Arg Asp

Trp Val Arg Try Asn Gln Phe Arg Arg Glu Leu Thr Leu Thr Val

Leu Asp Ile Val Ala Leu Phe Ser Asn Tyr Asp Ser Arg Arg Tyr

Pro Ile Arg Thr Val Ser Gln Leu Thr Arg Glu Ile Tyr Thr Asn

Pro Val leu Glu Asn Phe Asp Gly Ser Phe Arg Gly Met Ala Gln

Arg Ile Glu Gln Asn Ile Arg Gln Pro His Leu Met Asp Ile Leu

Asn Arg Ile Thr Ile Tyr Thr Asp Val His Arg Gly Phe Asn Tyr

Trp Ser Gly His Gln Ile Thr Ala Ser Pro Val Gly Phe Ser Gly

Pro Glu Phe Ala Phe Pro Leu Phe Gly Asn Ala Gly Asn Ala Ala

Pro Pro Val Leu Val Ser Leu Thr Gly Leu Gly Ile Phe Arg Thr

Leu Ser Ser Pro Leu Tyr Arg Arg Ile Ile Leu Gly Ser Gly Pro

Asn Asn Gln Glu Leu Phe Val Leu Asp Gly Thr Glu Phe Ser Phe

Ala Ser Leu Thr Thr Asn Leu Pro Ser Thr Ile Tyr Arg Gln Arg

Gly Thr Val Asp Ser Leu Asp Val Ile Pro Pro Gln Asp Asn Ser

Val Pro Pro Arg Ala Gly Phe Ser His Arg Leu Ser His Val Thr

Met Leu Ser Gln Ala Ala Gly Ala Val Tyr Thr Leu Arg Ala Pro

Thr Phe Ser Trp Gln His Arg Ser Ala Glu Phe Asn Asn Ile Ile

Pro Ser Ser Gln Ile Thr Gln Ile Pro Leu Thr Lys Ser Thr Asn

490
Leu Gly Ser Gly Thr Ser Val Val Lys Gly Pro Gly Phe Thr Gly

500                                                510
Gly Asp Ile Leu Arg Arg Thr Ser Pro Gly Gln Ile Ser Thr Leu

                              520
Arg Val Asn Ile Thr Ala Pro Leu Ser Gln Arg Tyr Arg Val Arg

530                                                540
Ile Arg Tyr Ala Ser Thr Thr Asn Leu Gln Phe His Thr Ser Ile

                              550
Asp Gly Arg Pro Ile Asn Gln Gly Asn Phe Ser Ala Thr Met Ser

560                                                570
Ser Gly Ser Asn Leu Gln Ser Gly Ser Phe Arg Thr Val Gly Phe

                              580
Thr Thr Pro Phe Asn Phe Ser Asn Gly Ser Ser Val Phe Thr Leu

590                                                600
Ser Ala His Val Phe Asn Ser Gly Asn Glu Val Tyr Ile Asp Arg

                              610
Ile Glu Phe Val Pro Ala Glu Val Thr Phe Glu Ala Glu Tyr Asp

620                                                630
Leu Glu Arg Ala Gln Lys Ala Val Asn Glu Leu Phe Thr Ser Ser

                              640
Asn Gln Ile Gly Leu Lys Thr Asp Val Thr Asp Tyr His Ile Asp

650                                                660
Gln Val Ser Asn Leu Val Glu Cys Leu Ser Asp Glu Phe Cys Leu

                              670
Asp Glu Lys Gln Glu Leu Ser Glu Lys Val Lys His Ala Lys Arg

680                                                690
Leu Ser Asp Glu Arg Asn Leu Leu Gln Asp Pro Asn Phe Arg Gly

                              700
Ile Asn Arg Gln Leu Asp Arg Gly Trp Arg Gly Ser Thr Asp Ile

710                                                720
Thr Ile Gln Gly Gly Asp Asp Val Phe Lys Glu Asn Tyr Val Thr

                              730
Leu Leu Gly Thr Phe Asp Glu Cys Tyr Pro Thr Tyr Leu Tyr Gln

740                                                750
Lys Ile Asp Glu Ser Lys Leu Lys Ala Tyr Thr Arg Tyr Gln Leu

11

760
Arg Gly Tyr Ile Glu Asp Ser Gln Asp Leu Glu Ile Tyr Leu Ile

770                                              780
Arg Tyr Asn Ala Lys His Glu Thr Val Asn Val Pro Gly Thr Gly

790
Ser Leu Trp Pro Leu Ser Ala Gln Ser Pro Ile Gly Lys Cys Gly

800                                              810
Glu Pro Asn Arg Cys Ala Pro His Leu Glu Trp Asn Pro Asp Leu

820
Asp Cys Ser Cys Arg Asp Gly Glu Lys Cys Ala His His Ser His

830                                              840
His Phe Ser Leu Asp Ile Asp Val Gly Cys Thr Asp Leu Asn Glu

850
Asp Leu Gly Val Trp Val Ile Phe Lys Ile Lys Thr Gln Asp Gly

860                                              870
His Ala Arg Leu Gly Asn Leu Glu Phe Leu Glu Glu Lys Pro Leu

880
Val Gly Glu Ala Leu Ala Arg Val Lys Arg Ala Glu Lys Lys Trp

890                                              900
Arg Asp Lys Arg Glu Lys Leu Glu Trp Glu Thr Asn Ile Val Tyr

910
Lys Glu Ala Lys Glu Ser Val Asp Ala Leu Phe Val Asn Ser Gln

920                                              930
Tyr Asp Arg Leu Gln Ala Asp Thr Asn Ile Ala Met Ile His Ala

940
Ala Asp Lys Arg Val His Ser Ile Arg Glu Ala Tyr Leu Pro Glu

950                                              960
Leu Ser Val Ile Pro Gly Val Asn Ala Ala Ile Phe Glu Glu Leu

970
Glu Gly Arg Ile Phe Thr Ala Phe Ser Leu Tyr Asp Ala Arg Asn

980                                              990
Val Ile Lys Asn Gly Asp Phe Asn Asn Gly Leu Ser Cys Trp Asn

1000
Val Lys Gly His Val Asp Val Glu Glu Gln Asn Asn His Arg Ser

1010                                             1020
Val Leu Val Val Pro Glu Trp Glu Ala Glu Val Ser Gln Glu Val

1030
Arg Val Cys Pro Gly Arg Gly Tyr Ile Leu Arg Val Thr Ala Tyr

1040                                          1050
Lys Glu Gly Tyr Gly Glu Gly Cys Val Thr Ile His Glu Ile Glu

1060
Asn Asn Thr Asp Glu Leu Lys Phe Ser Asn Cys Val Glu Glu Glu

1070                                          1080
Val Tyr Pro Asn Asn Thr Val Thr Cys Asn Asp Tyr Thr Ala Thr

1090
Gln Glu Glu Tyr Glu Gly Thr Tyr Thr Ser Arg Asn Arg Gly Tyr

1100                                          1110
Asp Gly Ala Tyr Glu Ser Asn Ser Ser Val Pro Ala Asp Tyr Ala

1120
Ser Ala Tyr Glu Glu Lys Ala Tyr Thr Asp Gly Arg Arg Asp Asn

1130                                          1140
Pro Cys Glu Ser Asn Arg Gly Tyr Gly Asp Tyr Thr Pro Leu Pro

1150
Ala Gly Tyr Val Thr Lys Glu Leu Glu Tyr Phe Pro Glu Thr Asp

1160                                          1170
Lys Val Trp Ile Glu Ile Gly Glu Thr Glu Gly Thr Phe Ile Val

1180
Asp Ser Val Glu Leu Leu Leu Met Glu Glu,

or having an amino acid sequence comprising at least 723 amino acids from the N-terminus of said amino acid sequence.

2. A DNA fragment according to claim 1, wherein the nucleotide sequence comprises the following sequence:

ATG GAT AAC AAT CCG AAC ATC AAT GAA TGC ATT CCT TAT AAT TGT

TTA AGT AAC CCT GAA GTA GAA GTA TTA GGT GGA GAA AGA GTG GAA   242

ACT GGT TAC ACC CCA ATC GAT ATT TCC TTG TCG CTA ACG CAA TTT

CTT TTG AGT GAA TTT GTT CCC GGT GCT GGA TTT GTG TTA GGA CTA   332

GTT GAT ATA ATA TGG GGA ATT TTT GGT CCC TCT CAA TGG GAC GCA

TTT CTT GTA CAA ATT GAA CAG TTA ATT AAC CAA AGA ATA GAA GAA   422

TTC GCT AGG AAC CAA GCC ATT TCT AGA TTA GAA GGA CTA AGC AAT

CTT TAT CAA ATT TAC GCA GAA TCT TTT AGA GAG TGG GAA GCA GAT   512

CCT ACT AAT CCA GCA TTA AGA GAA GAG ATG CGT ATT CAA TTC AAT

GAC ATG AAC AGT GCC CTT ACA ACC GCT ATT CCT CTT TTT GCA GTT   602

CAA AAT TAT CAA GTT CCT CTT TTA TCA GTA TAT GTT CAA GCT GCA

AAT TTA CAT TTA TCA GTT TTG AGA GAT GTT TCA GTG TTT GGA CAA   692

AGG TGG GGA TTT GAT GCC GCG ACT ATC AAT AGT CGT TAT AAT GAT

TTA ACT AGG CTT ATT GGC AAC TAT ACA GAT TAT GCT GTG CGC TGG   782

TAC AAT ACG GGA TTA GAG CGT GTA TGG GGA CCG GAT TCT AGA GAT

TGG GTA AGG TAT AAT CAA TTT AGA AGA GAG CTA ACA CTT ACT GTA   872

TTA GAT ATC CTT GCT CTA TTC TCA AAT TAT GAT AGT CGA AGG TAT

CCA ATT CGA ACA GTT TCC CAA TTA ACA AGA GAA ATT TAT ACG AAC   962

CCA GTA TTA GAA AAT TTT GAT GGT AGT TTT CGT GGA ATG GCT CAG

AGA ATA GAA CAG AAT ATT AGG CAA CCA CAT CTT ATG GAT ATC CTT   1052

AAT AGG ATA ACC ATT TAT ACT GAT GTG CAT AGA GGC TTT AAT TAT

TGG TCA GGG CAT CAA ATA ACA GCT TCT CCT GTA GGG TTT TCA GGA   1142

CCA GAA TTC GCA TTC CCT TTA TTT GGG AAT GCG GGG AAT GCA GCT

CCA CCC GTA CTT GTC TCA TTA ACT GGT TTG GGG ATT TTT AGA ACA   1232

TTA TCT TCA CCT TTA TAT AGA AGA ATT ATA CTT GGT TCA GGC CCA

AAT AAT CAG GAA CTG TTT GTC CTT GAT GGA ACG GAG TTT TCT TTT   1322

GCC TCC CTA ACG ACC AAC TTG CCT TCC ACT ATA TAT AGA CAA AGG

GGT ACA GTC GAT TCA CTA GAT GTA ATA CCG CCA CAG GAT AAT AGT   1412

GTA CCA CCT CGT GCG GGA TTT AGC CAT CGA TTG AGT CAT GTT ACA

ATG CTG AGC CAA GCA GCT GGA GCA GTT TAC ACC TTG AGA GCT CCA   1502

ACG TTT TCT TGG CAG CAT CGC AGT GCT GAA TTT AAT AAT ATA ATT

CCT TCA TCA CAA ATT ACA CAA ATA CCT TTA ACA AAA TCT ACT AAT   1592

CTT GGC TCT GGA ACT TCT GTC GTT AAA GGA CCA GGA TTT ACA GGA

GGA GAT ATT CTT CGA AGA ACT TCA CCT GGC CAG ATT TCA ACC TTA   1682

AGA GTA AAT ATT ACT GCA CCA TTA TCA CAA AGA TAT CGG GTA AGA

ATT CGC TAC GCT TCT ACT ACA AAT TTA CAA TTC CAT ACA TCA ATT   1772

GAC GGA AGA CCT ATT AAT CAG GGT AAT TTT TCA GCA ACT ATG AGT

```
AGT GGG AGT AAT TTA CAG TCC GGA AGC TTT AGG ACT GTA GGT TTT  1862
ACT ACT CCG TTT AAC TTT TCA AAT GGA TCA AGT GTA TTT ACG TTA
AGT GCT CAT GTC TTC AAT TCA GGC AAT GAA GTT TAT ATA GAT CGA  1952
ATT GAA TTT GTT CCG GCA GAA GTA ACC TTT GAG GCA GAA TAT GAT
TTA GAA AGA GCA CAA AAG GCG GTG AAT GAG CTG TTT ACT TCT TCC  2042
AAT CAA ATC GGG TTA AAA ACA GAT GTG ACG GAT TAT CAT ATT GAT
CAA GTA TCC AAT TTA GTT GAG TGT TTA TCA GAT GAA TTT TGT CTG  2132
GAT GAA AAA CAA GAA TTG TCC GAG AAA GTC AAA CAT GCG AAG CGA
CTT AGT GAT GAG CGG AAT TTA CTT CAA GAT CCA AAC TTC AGA GGG  2222
ATC AAT AGA CAA CTA GAC CGT GGC TGG AGA GGA AGT ACG GAT ATT
ACC ATC CAA GGA GGC GAT GAC GTA TTC AAA GAG AAT TAC GTT ACG  2312
CTA TTG GGT ACC TTT GAT GAG TGC TAT CCA ACG TAT TTA TAT CAA
AAA ATA GAT GAG TCG AAA TTA AAA GCC TAT ACC CGT TAT CAA TTA  2402
AGA GGG TAT ATC GAA GAT AGT CAA GAC TTA GAA ATC TAT TTA ATT
CGC TAC AAT GCA AAA CAT GAA ACA GTA AAT GTG CCA GGT ACG GGT  2492
TCC TTA TGG CCG CTT TCA GCC CAA AGT CCA ATC GGA AAG TGT GGA
GAG CCG AAT CGA TGC GCG CCA CAC CTT GAA TGG AAT CCT GAC TTA  2582
GAT TGT TCG TGT AGG GAT GGA GAA AAA TGT GCC CAT CAT TCC CAT
CAT TTC TCC TTG GAC ATT GAT GTT GGA TGT ACA GAC TTA AAT GAG  2672
GAC TTA GGT GTA TGG GTG ATA TTC AAG ATT AAG ACG CAA GAT GGC
CAT GCA AGA CTA GGA AAT CTA GAA TTT CTC GAA GAG AAA CCA TTA  2762
GTA GGA GAA GCA CTA GCT CGT GTG AAA AGA GCC GAG AAA AAA TGG
AGA GAC AAA CGT GAA AAA TTG GAA TGG GAA ACA AAT ATT GTT TAT  2852
AAA GAG GCA AAA GAA TCT GTA GAT GCT TTA TTT GTA AAC TCT CAA
TAT GAT AGA TTA CAA GCG GAT ACC AAC ATC GCG ATG ATT CAT GCG  2942
GCA GAT AAA CGC GTT CAT AGC ATT CGA GAA GCT TAT CTG CCT GAG
CTG TCT GTG ATT CCG GGT GTC AAT GCG GCT ATT TTT GAA GAA TTA  3032
GAA GGG CGT ATT TTC ACT GCA TTC TCC CTA TAT GAT GCG AGA AAT
```

16

GTC ATT AAA AAT GGT GAT TTT AAT AAT GGC TTA TCC TGC TGG AAC  3122

GTG AAA GGG CAT GTA GAT GTA GAA GAA CAA AAC AAC CAC CGT TCG

GTC CTT GTT GTT CCG GAA TGG GAA GCA GAA GTG TCA CAA GAA GTT  3212

CGT GTC TGT CCG GGT CGT GGC TAT ATC CTT CGT GTC ACA GCG TAC

AAG GAG GGA TAT GGA GAA GGT TGC GTA ACC ATT CAT GAG ATC GAG  3302

AAC AAT ACA GAC GAA CTG AAG TTT AGC AAC TGT GTA GAA GAG GAA

GTA TAT CCA AAC AAC ACG GTA ACG TGT AAT GAT TAT ACT GCG ACT  3392

CAA GAA GAA TAT GAG GGT ACG TAC ACT TCT CGT AAT CGA GGA TAT

GAC GGA GCC TAT GAA AGC AAT TCT TCT GTA CCA GCT GAT TAT GCA  3482

TCA GCC TAT GAA GAA AAA GCA TAT ACA GAT GGA CGA AGA GAC AAT

CCT TGT GAA TCT AAC AGA GGA TAT GGG GAT TAC ACA CCA CTA CCA  3572

GCT GGC TAT GTG ACA AAA GAA TTA GAG TAC TTC CCA GAA ACC GAT

AAG GTA TGG ATT GAG ATC GGA GAA ACG GAA GGA ACA TTC ATC GTG  3662

GAC AGC GTG GAA TTA CTT CTT ATG GAG GAA,

or a sequence comprising at least 723 codons from the 5'-terminus of said nucleotide sequence.

3. A DNA fragment according to claim 1 or claim 2, about 6.6 kb in length.

4. A DNA fragment according to claim 1, wherein the amino acid sequence encoded by the nucleotide sequence comprises an amino acid sequence starting from Met at the amino acid position 1 and terminating at Arg at the amino acid position 934 defined in claim 1.

5. A DNA fragment according to claim 4, wherein the nucleotide sequence coding for the amino acid sequence comprises a nucleotide sequence starting from the position 153 and terminating at the position 2955 defined in claim 2.

6. A DNA fragment according to any one of claims 1, 4 and 5, about 2.95 kb in length.

7. A plasmid capable of replicating in E. coli and expressing the peptide defined in claim 1, comprising a plasmid pBR322 in which the DNA sequence defined in claim 1 has been inserted.

8. A plasmid according to claim 7, wherein the inserted DNA fragment is about 6.6 kb in length.

9. A plasmid according to claim 8, wherein the DNA fragment has been inserted at Pst I cleavage site in plasmid pBR322.

10. A plasmid according to claim 7, wherein the inserted DNA fragment is about 2.95 kb in length.

11. A plasmid according to claim 10, wherein the DNA fragment has been inserted at Bam HI cleavage site in plasmid pBR322.

12. E. coli transformed with a plasmid according to any one of claims 7 to 11, and producing an insecticidal protein.

13. E. coli according to claim 12, wherein the protein has a molecular weight of 144 kD.

14. E. coli according to claim 12, wherein the protein has a molecular weight of 75 kD.

15. An insecticidal protein which has been produced by an organism transformed so as to incorporate a DNA fragment according to any one of claims 1 to 6.

16. A composition comprising an insecticidally effective amount of protein according to claim 15, together with a carrier.

17. A process for making an insecticidal protein, comprising production of said protein by an organism transformed so as to incorporate a DNA fragment according to any one of claims 1 to 6.


CLAIMS: [ FOR THE CONTRACTING STATE AT]

1. A process comprising preparation of a DNA fragment comprising a nucleotide sequence coding for an insecticidal peptide having the following amino acid sequence:

```
    1                                          10
Met Asp Asn Asn Pro Asn Ile Asn Glu Cys Ile Pro Tyr Asn Cys

                 20                                          30
Leu Ser Asn Pro Glu Val Glu Val Leu Gly Gly Glu Arg Ile Glu

                                          40
Thr Gly Tyr Thr Pro Ile Asp Ile Ser Leu Ser Leu Thr Gln Phe

                 50                                          60
Leu Leu Ser Glu Phe Val Pro Gly Ala Gly Phe Val Leu Gly Leu

                                     70
Val Asp Ile Ile Trp Gly Ile Phe Gly Pro Ser Gln Trp Asp Ala

                 80                                          90
Phe Leu Val Gln Ile Glu Gln Leu Ile Asn Gln Arg Ile Glu Glu

                               100
Phe Ala Arg Asn Gln Ala Ile Ser Arg Leu Glu Gly Leu Ser Asn

                 110                                        120
Leu Tyr Gln Ile Tyr Ala Glu Ser Phe Arg Glu Trp Glu Ala Asp

                               130
Pro Thr Asn Pro Ala Leu Arg Glu Glu Met Arg Ile Gln Phe Asn

                 140                                        150
Asp Met Asn Ser Ala Leu Thr Thr Ala Ile Pro Leu Phe Ala Val

                               160
Gln Asn Tyr Gln Val Pro Leu Leu Ser Val Tyr Val Gln Ala Ala

                 170                                        180
Asn Leu His Leu Ser Val Leu Arg Asp Val Ser Val Phe Gly Gln

                               190
Arg Trp Gly Phe Asp Ala Ala Thr Ile Asn Ser Arg Tyr Asn Asp

                 200                                        210
Leu Thr Arg Leu Ile Gly Asn Tyr Thr Asp Tyr Ala Val Arg Trp
```

220
Tyr Asn Thr Gly Leu Glu Arg Val Trp Gly Pro Asp Ser Arg Asp

230                                                         240
Trp Val Arg Try Asn Gln Phe Arg Arg Glu Leu Thr Leu Thr Val

250
Leu Asp Ile Val Ala Leu Phe Ser Asn Tyr Asp Ser Arg Arg Tyr

260                                                         270
Pro Ile Arg Thr Val Ser Gln Leu Thr Arg Glu Ile Tyr Thr Asn

280
Pro Val leu Glu Asn Phe Asp Gly Ser Phe Arg Gly Met Ala Gln

290                                                         300
Arg Ile Glu Gln Asn Ile Arg Gln Pro His Leu Met Asp Ile Leu

310
Asn Arg Ile Thr Ile Tyr Thr Asp Val His Arg Gly Phe Asn Tyr

320                                                         330
Trp Ser Gly His Gln Ile Thr Ala Ser Pro Val Gly Phe Ser Gly

340
Pro Glu Phe Ala Phe Pro Leu Phe Gly Asn Ala Gly Asn Ala Ala

350                                                         360
Pro Pro Val Leu Val Ser Leu Thr Gly Leu Gly Ile Phe Arg Thr

370
Leu Ser Ser Pro Leu Tyr Arg Arg Ile Ile Leu Gly Ser Gly Pro

380                                                         390
Asn Asn Gln Glu Leu Phe Val Leu Asp Gly Thr Glu Phe Ser Phe

400
Ala Ser Leu Thr Thr Asn Leu Pro Ser Thr Ile Tyr Arg Gln Arg

410                                                         420
Gly Thr Val Asp Ser Leu Asp Val Ile Pro Pro Gln Asp Asn Ser

430
Val Pro Pro Arg Ala Gly Phe Ser His Arg Leu Ser His Val Thr

440                                                         450
Met Leu Ser Gln Ala Ala Gly Ala Val Tyr Thr Leu Arg Ala Pro

460
Thr Phe Ser Trp Gln His Arg Ser Ala Glu Phe Asn Asn Ile Ile

470                                                         480
Pro Ser Ser Gln Ile Thr Gln Ile Pro Leu Thr Lys Ser Thr Asn

490
Leu Gly Ser Gly Thr Ser Val Val Lys Gly Pro Gly Phe Thr Gly

500                                             510
Gly Asp Ile Leu Arg Arg Thr Ser Pro Gly Gln Ile Ser Thr Leu

520
Arg Val Asn Ile Thr Ala Pro Leu Ser Gln Arg Tyr Arg Val Arg

530                                             540
Ile Arg Tyr Ala Ser Thr Thr Asn Leu Gln Phe His Thr Ser Ile

550
Asp Gly Arg Pro Ile Asn Gln Gly Asn Phe Ser Ala Thr Met Ser

560                                             570
Ser Gly Ser Asn Leu Gln Ser Gly Ser Phe Arg Thr Val Gly Phe

580
Thr Thr Pro Phe Asn Phe Ser Asn Gly Ser Ser Val Phe Thr Leu

590                                             600
Ser Ala His Val Phe Asn Ser Gly Asn Glu Val Tyr Ile Asp Arg

610
Ile Glu Phe Val Pro Ala Glu Val Thr Phe Glu Ala Glu Tyr Asp

620                                             630
Leu Glu Arg Ala Gln Lys Ala Val Asn Glu Leu Phe Thr Ser Ser

640
Asn Gln Ile Gly Leu Lys Thr Asp Val Thr Asp Tyr His Ile Asp

650                                             660
Gln Val Ser Asn Leu Val Glu Cys Leu Ser Asp Glu Phe Cys Leu

670
Asp Glu Lys Gln Glu Leu Ser Glu Lys Val Lys His Ala Lys Arg

680                                             690
Leu Ser Asp Glu Arg Asn Leu Leu Gln Asp Pro Asn Phe Arg Gly

700
Ile Asn Arg Gln Leu Asp Arg Gly Trp Arg Gly Ser Thr Asp Ile

710                                             720
Thr Ile Gln Gly Gly Asp Asp Val Phe Lys Glu Asn Tyr Val Thr

730
Leu Leu Gly Thr Phe Asp Glu Cys Tyr Pro Thr Tyr Leu Tyr Gln

740                                             750
Lys Ile Asp Glu Ser Lys Leu Lys Ala Tyr Thr Arg Tyr Gln Leu

21

760
Arg Gly Tyr Ile Glu Asp Ser Gln Asp Leu Glu Ile Tyr Leu Ile

770                                         780
Arg Tyr Asn Ala Lys His Glu Thr Val Asn Val Pro Gly Thr Gly

790
Ser Leu Trp Pro Leu Ser Ala Gln Ser Pro Ile Gly Lys Cys Gly

800                                         810
Glu Pro Asn Arg Cys Ala Pro His Leu Glu Trp Asn Pro Asp Leu

820
Asp Cys Ser Cys Arg Asp Gly Glu Lys Cys Ala His His Ser His

830                                         840
His Phe Ser Leu Asp Ile Asp Val Gly Cys Thr Asp Leu Asn Glu

850
Asp Leu Gly Val Trp Val Ile Phe Lys Ile Lys Thr Gln Asp Gly

860                                         870
His Ala Arg Leu Gly Asn Leu Glu Phe Leu Glu Glu Lys Pro Leu

880
Val Gly Glu Ala Leu Ala Arg Val Lys Arg Ala Glu Lys Lys Trp

890                                         900
Arg Asp Lys Arg Glu Lys Leu Glu Trp Glu Thr Asn Ile Val Tyr

910
Lys Glu Ala Lys Glu Ser Val Asp Ala Leu Phe Val Asn Ser Gln

920                                         930
Tyr Asp Arg Leu Gln Ala Asp Thr Asn Ile Ala Met Ile His Ala

940
Ala Asp Lys Arg Val His Ser Ile Arg Glu Ala Tyr Leu Pro Glu

950                                         960
Leu Ser Val Ile Pro Gly Val Asn Ala Ala Ile Phe Glu Glu Leu

970
Glu Gly Arg Ile Phe Thr Ala Phe Ser Leu Tyr Asp Ala Arg Asn

980                                         990
Val Ile Lys Asn Gly Asp Phe Asn Asn Gly Leu Ser Cys Trp Asn

1000
Val Lys Gly His Val Asp Val Glu Glu Gln Asn Asn His Arg Ser

1010                                        1020
Val Leu Val Val Pro Glu Trp Glu Ala Glu Val Ser Gln Glu Val

1030
Arg Val Cys Pro Gly Arg Gly Tyr Ile Leu Arg Val Thr Ala Tyr

1040                                              1050
Lys Glu Gly Tyr Gly Glu Gly Cys Val Thr Ile His Glu Ile Glu

1060
Asn Asn Thr Asp Glu Leu Lys Phe Ser Asn Cys Val Glu Glu Glu

1070                                              1080
Val Tyr Pro Asn Asn Thr Val Thr Cys Asn Asp Tyr Thr Ala Thr

1090
Gln Glu Glu Tyr Glu Gly Thr Tyr Thr Ser Arg Asn Arg Gly Tyr

1100                                              1110
Asp Gly Ala Tyr Glu Ser Asn Ser Ser Val Pro Ala Asp Tyr Ala

1120
Ser Ala Tyr Glu Glu Lys Ala Tyr Thr Asp Gly Arg Arg Asp Asn

1130                                              1140
Pro Cys Glu Ser Asn Arg Gly Tyr Gly Asp Tyr Thr Pro Leu Pro

1150
Ala Gly Tyr Val Thr Lys Glu Leu Glu Tyr Phe Pro Glu Thr Asp

1160                                              1170
Lys Val Trp Ile Glu Ile Gly Glu Thr Glu Gly Thr Phe Ile Val

1180
Asp Ser Val Glu Leu Leu Leu Met Glu Glu,

or having an amino acid sequence comprising at least 723 amino acids from the N-terminus of said amino acid sequence.

2. A process according to claim 1, wherein the nucleotide sequence comprises the following sequence:

ATG GAT AAC AAT CCG AAC ATC AAT GAA TGC ATT CCT TAT AAT TGT

TTA AGT AAC CCT GAA GTA GAA GTA TTA GGT GGA GAA AGA GTG GAA    242

ACT GGT TAC ACC CCA ATC GAT ATT TCC TTG TCG CTA ACG CAA TTT

CTT TTG AGT GAA TTT GTT CCC GGT GCT GGA TTT GTG TTA GGA CTA    332

GTT GAT ATA ATA TGG GGA ATT TTT GGT CCC TCT CAA TGG GAC GCA

TTT CTT GTA CAA ATT GAA CAG TTA ATT AAC CAA AGA ATA GAA GAA    422

TTC GCT AGG AAC CAA GCC ATT TCT AGA TTA GAA GGA CTA AGC AAT

CTT TAT CAA ATT TAC GCA GAA TCT TTT AGA GAG TGG GAA GCA GAT    512

CCT ACT AAT CCA GCA TTA AGA GAA GAG ATG CGT ATT CAA TTC AAT

GAC ATG AAC AGT GCC CTT ACA ACC GCT ATT CCT CTT TTT GCA GTT  602

CAA AAT TAT CAA GTT CCT CTT TTA TCA GTA TAT GTT CAA GCT GCA

AAT TTA CAT TTA TCA GTT TTG AGA GAT GTT TCA GTG TTT GGA CAA  692

AGG TGG GGA TTT GAT GCC GCG ACT ATC AAT AGT CGT TAT AAT GAT

TTA ACT AGG CTT ATT GGC AAC TAT ACA GAT TAT GCT GTG CGC TGG  782

TAC AAT ACG GGA TTA GAG CGT GTA TGG GGA CCG GAT TCT AGA GAT

TGG GTA AGG TAT AAT CAA TTT AGA AGA GAG CTA ACA CTT ACT GTA  872

TTA GAT ATC CTT GCT CTA TTC TCA AAT TAT GAT AGT CGA AGG TAT

CCA ATT CGA ACA GTT TCC CAA TTA ACA AGA GAA ATT TAT ACG AAC  962

CCA GTA TTA GAA AAT TTT GAT GGT AGT TTT CGT GGA ATG GCT CAG

AGA ATA GAA CAG AAT ATT AGG CAA CCA CAT CTT ATG GAT ATC CTT  1052

AAT AGG ATA ACC ATT TAT ACT GAT GTG CAT AGA GGC TTT AAT TAT

TGG TCA GGG CAT CAA ATA ACA GCT TCT CCT GTA GGG TTT TCA GGA  1142

CCA GAA TTC GCA TTC CCT TTA TTT GGG AAT GCG GGG AAT GCA GCT

CCA CCC GTA CTT GTC TCA TTA ACT GGT TTG GGG ATT TTT AGA ACA  1232

TTA TCT TCA CCT TTA TAT AGA AGA ATT ATA CTT GGT TCA GGC CCA

AAT AAT CAG GAA CTG TTT GTC CTT GAT GGA ACG GAG TTT TCT TTT  1322

GCC TCC CTA ACG ACC AAC TTG CCT TCC ACT ATA TAT AGA CAA AGG

GGT ACA GTC GAT TCA CTA GAT GTA ATA CCG CCA CAG GAT AAT AGT  1412

GTA CCA CCT CGT GCG GGA TTT AGC CAT CGA TTG AGT CAT GTT ACA

ATG CTG AGC CAA GCA GCT GGA GCA GTT TAC ACC TTG AGA GCT CCA  1502

ACG TTT TCT TGG CAG CAT CGC AGT GCT GAA TTT AAT AAT ATA ATT

CCT TCA TCA CAA ATT ACA CAA ATA CCT TTA ACA AAA TCT ACT AAT  1592

CTT GGC TCT GGA ACT TCT GTC GTT AAA GGA CCA GGA TTT ACA GGA

GGA GAT ATT CTT CGA AGA ACT TCA CCT GGC CAG ATT TCA ACC TTA  1682

AGA GTA AAT ATT ACT GCA CCA TTA TCA CAA AGA TAT CGG GTA AGA

ATT CGC TAC GCT TCT ACT ACA AAT TTA CAA TTC CAT ACA TCA ATT  1772

GAC GGA AGA CCT ATT AAT CAG GGT AAT TTT TCA GCA ACT ATG AGT

```
AGT GGG AGT AAT TTA CAG TCC GGA AGC TTT AGG ACT GTA GGT TTT   1862

ACT ACT CCG TTT AAC TTT TCA AAT GGA TCA AGT GTA TTT ACG TTA

AGT GCT CAT GTC TTC AAT TCA GGC AAT GAA GTT TAT ATA GAT CGA   1952

ATT GAA TTT GTT CCG GCA GAA GTA ACC TTT GAG GCA GAA TAT GAT

TTA GAA AGA GCA CAA AAG GCG GTG AAT GAG CTG TTT ACT TCT TCC   2042

AAT CAA ATC GGG TTA AAA ACA GAT GTG ACG GAT TAT CAT ATT GAT

CAA GTA TCC AAT TTA GTT GAG TGT TTA TCA GAT GAA TTT TGT CTG   2132

GAT GAA AAA CAA GAA TTG TCC GAG AAA GTC AAA CAT GCG AAG CGA

CTT AGT GAT GAG CGG AAT TTA CTT CAA GAT CCA AAC TTC AGA GGG   2222

ATC AAT AGA CAA CTA GAC CGT GGC TGG AGA GGA AGT ACG GAT ATT

ACC ATC CAA GGA GGC GAT GAC GTA TTC AAA GAG AAT TAC GTT ACG   2312

CTA TTG GGT ACC TTT GAT GAG TGC TAT CCA ACG TAT TTA TAT CAA

AAA ATA GAT GAG TCG AAA TTA AAA GCC TAT ACC CGT TAT CAA TTA   2402

AGA GGG TAT ATC GAA GAT AGT CAA GAC TTA GAA ATC TAT TTA ATT

CGC TAC AAT GCA AAA CAT GAA ACA GTA AAT GTG CCA GGT ACG GGT   2492

TCC TTA TGG CCG CTT TCA GCC CAA AGT CCA ATC GGA AAG TGT GGA

GAG CCG AAT CGA TGC GCG CCA CAC CTT GAA TGG AAT CCT GAC TTA   2582

GAT TGT TCG TGT AGG GAT GGA GAA AAA TGT GCC CAT CAT TCC CAT

CAT TTC TCC TTG GAC ATT GAT GTT GGA TGT ACA GAC TTA AAT GAG   2672

GAC TTA GGT GTA TGG GTG ATA TTC AAG ATT AAG ACG CAA GAT GGC

CAT GCA AGA CTA GGA AAT CTA GAA TTT CTC GAA GAG AAA CCA TTA   2762

GTA GGA GAA GCA CTA GCT CGT GTG AAA AGA GCC GAG AAA AAA TGG

AGA GAC AAA CGT GAA AAA TTG GAA TGG GAA ACA AAT ATT GTT TAT   2852

AAA GAG GCA AAA GAA TCT GTA GAT GCT TTA TTT GTA AAC TCT CAA

TAT GAT AGA TTA CAA GCG GAT ACC AAC ATC GCG ATG ATT CAT GCG   2942

GCA GAT AAA CGC GTT CAT AGC ATT CGA GAA GCT TAT CTG CCT GAG

CTG TCT GTG ATT CCG GGT GTC AAT GCG GCT ATT TTT GAA GAA TTA   3032

GAA GGG CGT ATT TTC ACT GCA TTC TCC CTA TAT GAT GCG AGA AAT
```

```
GTC ATT AAA AAT GGT GAT TTT AAT AAT GGC TTA TCC TGC TGG AAC   3122

GTG AAA GGG CAT GTA GAT GTA GAA GAA CAA AAC AAC CAC CGT TCG

GTC CTT GTT GTT CCG GAA TGG GAA GCA GAA GTG TCA CAA GAA GTT   3212

CGT GTC TGT CCG GGT CGT GGC TAT ATC CTT CGT GTC ACA GCG TAC

AAG GAG GGA TAT GGA GAA GGT TGC GTA ACC ATT CAT GAG ATC GAG   3302

AAC AAT ACA GAC GAA CTG AAG TTT AGC AAC TGT GTA GAA GAG GAA

GTA TAT CCA AAC AAC ACG GTA ACG TGT AAT GAT TAT ACT GCG ACT   3392

CAA GAA GAA TAT GAG GGT ACG TAC ACT TCT CGT AAT CGA GGA TAT

GAC GGA GCC TAT GAA AGC AAT TCT TCT GTA CCA GCT GAT TAT GCA   3482

TCA GCC TAT GAA GAA AAA GCA TAT ACA GAT GGA CGA AGA GAC AAT

CCT TGT GAA TCT AAC AGA GGA TAT GGG GAT TAC ACA CCA CTA CCA   3572

GCT GGC TAT GTG ACA AAA GAA TTA GAG TAC TTC CCA GAA ACC GAT

AAG GTA TGG ATT GAG ATC GGA GAA ACG GAA GGA ACA TTC ATC GTG   3662

GAC AGC GTG GAA TTA CTT CTT ATG GAG GAA,
```

or a sequence comprising at least 723 codons from the 5'-terminus of said nucleotide sequence.

3. A process according to claim 1 or claim 2, wherein the DNA fragment is about 6.6 kb in length.

4. A process according to claim 1, wherein the amino acid sequence encoded by the nucleotide sequence comprises an amino acid sequence starting from Met at the amino acid position 1 and terminating at Arg at the amino acid position 934 defined in claim 1.

5. A process according to claim 4, wherein the nucleotide sequence coding for the amino acid sequence comprises a nucleotide sequence starting from the position 153 and terminating at the position 2955 defined in claim 2.

6. A process according to claims 1, 4 and 5, where the DNA fragment is about 2.95 kb in length.

7. A process comprising preparation of a plasmid capable of replicating in E.coli and expresing the peptide defined in claim 1, comprising insertion of a DNA sequence as defined in claim 1 in a plasmid pBR322.

8. A process according to claim 7, wherein the inserted DNA fragment is about 6.6 kb in length.

9. A process according to claim, 8 wherein the DNA fragment is inserted at Pst I cleavage site in the plasmid pBR322.

10. A process according to claim 7, wherein the inserted DNA fragment is about 2.95 kb in length.

11. A process according to claim 10, wherein the DNA fragment is inserted at Bam HI cleavage site in

EP 0 186 379 B1

plasmid pBR322.

12. E. coli transformed with a plasmid prepared in accordance with any one of claims 7 to 11, and producing an insecticidal protein.

13. E. coli according to claim 12, wherein the protein has a molecular weight of 144 kD.

14. E. coli according to claim 12, wherein the protein has a molecular weight of 75 kD.

15. A process for making an insecticidal protein, comprising production of said protein by an organism transformed so as to incorporate a DNA fragment according to any one of claims 1 to 6.

16. A process according to claim 15 wherein said organism is E. coli according to any one of claims 12, 13 and 14.

**Revendications**

1. Un fragment d'ADN comprenant une séquence de nucléotide codant pour un peptide insecticide renfermant la séquence suivante d'amino-acide:

```
          1                                            10
Met Asp Asn Asn Pro Asn Ile Asn Glu Cys Ile Pro Tyr Asn Cys

                    20                                          30
Leu Ser Asn Pro Glu Val Glu Val Leu Gly Gly Glu Arg Ile Glu

                              40
Thr Gly Tyr Thr Pro Ile Asp Ile Ser Leu Ser Leu Thr Gln Phe

                    50                                          60
Leu Leu Ser Glu Phe Val Pro Gly Ala Gly Phe Val Leu Gly Leu

                              70
Val Asp Ile Ile Trp Gly Ile Phe Gly Pro Ser Gln Trp Asp Ala

                    80                                          90
Phe Leu Val Gln Ile Glu Gln Leu Ile Asn Gln Arg Ile Glu Glu

                              100
Phe Ala Arg Asn Gln Ala Ile Ser Arg Leu Glu Gly Leu Ser Asn

                    110                                         120
Leu Tyr Gln Ile Tyr Ala Glu Ser Phe Arg Glu Trp Glu Ala Asp

                              130
Pro Thr Asn Pro Ala Leu Arg Glu Glu Met Arg Ile Gln Phe Asn

                    140                                         150
Asp Met Asn Ser Ala Leu Thr Thr Ala Ile Pro Leu Phe Ala Val

                              160
Gln Asn Tyr Gln Val Pro Leu Leu Ser Val Tyr Val Gln Ala Ala

                    170                                         180
Asn Leu His Leu Ser Val Leu Arg Asp Val Ser Val Phe Gly Gln

                              190
Arg Trp Gly Phe Asp Ala Ala Thr Ile Asn Ser Arg Tyr Asn Asp

                    200                                         210
Leu Thr Arg Leu Ile Gly Asn Tyr Thr Asp Tyr Ala Val Arg Trp

                              220
Tyr Asn Thr Gly Leu Glu Arg Val Trp Gly Pro Asp Ser Arg Asp

                    230                                         240
Trp Val Arg Try Asn Gln Phe Arg Arg Glu Leu Thr Leu Thr Val
```

Leu Asp Ile Val Ala Leu Phe Ser Asn Tyr Asp Ser Arg Arg Tyr
250

Pro Ile Arg Thr Val Ser Gln Leu Thr Arg Glu Ile Tyr Thr Asn
260 270

Pro Val Leu Glu Asn Phe Asp Gly Ser Phe Arg Gly Met Ala Gln
280

Arg Ile Glu Gln Asn Ile Arg Gln Pro His Leu Met Asp Ile Leu
290 300

Asn Arg Ile Thr Ile Tyr Thr Asp Val His Arg Gly Phe Asn Tyr
310

Trp Ser Gly His Gln Ile Thr Ala Ser Pro Val Gly Phe Ser Gly
320 330

Pro Glu Phe Ala Phe Pro Leu Phe Gly Asn Ala Gly Asn Ala Ala
340

Pro Pro Val Leu Val Ser Leu Thr Gly Leu Gly Ile Phe Arg Thr
350 360

Leu Ser Ser Pro Leu Tyr Arg Arg Ile Ile Leu Gly Ser Gly Pro
370

Asn Asn Gln Glu Leu Phe Val Leu Asp Gly Thr Glu Phe Ser Phe
380 390

Ala Ser Leu Thr Thr Asn Leu Pro Ser Thr Ile Tyr Arg Gln Arg
400

Gly Thr Val Asp Ser Leu Asp Val Ile Pro Pro Gln Asp Asn Ser
410 420

Val Pro Pro Arg Ala Gly Phe Ser His Arg Leu Ser His Val Thr
430

Met Leu Ser Gln Ala Ala Gly Ala Val Tyr Thr Leu Arg Ala Pro
440 450

Thr Phe Ser Trp Gln His Arg Ser Ala Glu Phe Asn Asn Ile Ile
460

Pro Ser Ser Gln Ile Thr Gln Ile Pro Leu Thr Lys Ser Thr Asn
470 480

Leu Gly Ser Gly Thr Ser Val Val Lys Gly Pro Gly Phe Thr Gly
490

Gly Asp Ile Leu Arg Arg Thr Ser Pro Gly Gln Ile Ser Thr Leu
500 510

Arg Val Asn Ile Thr Ala Pro Leu Ser Gln Arg Tyr Arg Val Arg
520

530                                              540
Ile Arg Tyr Ala Ser Thr Thr Asn Leu Gln Phe His Thr Ser Ile

550
Asp Gly Arg Pro Ile Asn Gln Gly Asn Phe Ser Ala Thr Met Ser

560                                              570
Ser Gly Ser Asn Leu Gln Ser Gly Ser Phe Arg Thr Val Gly Phe

580
Thr Thr Pro Phe Asn Phe Ser Asn Gly Ser Ser Val Phe Thr Leu

590                                              600
Ser Ala His Val Phe Asn Ser Gly Asn Glu Val Tyr Ile Asp Arg

610
Ile Glu Phe Val Pro Ala Glu Val Thr Phe Glu Ala Glu Tyr Asp

620                                              630
Leu Glu Arg Ala Gln Lys Ala Val Asn Glu Leu Phe Thr Ser Ser

640
Asn Gln Ile Gly Leu Lys Thr Asp Val Thr Asp Tyr His Ile Asp

650                                              660
Gln Val Ser Asn Leu Val Glu Cys Leu Ser Asp Glu Phe Cys Leu

670
Asp Glu Lys Gln Glu Leu Ser Glu Lys Val Lys His Ala Lys Arg

680                                              690
Leu Ser Asp Glu Arg Asn Leu Leu Gln Asp Pro Asn Phe Arg Gly

700
Ile Asn Arg Gln Leu Asp Arg Gly Trp Arg Gly Ser Thr Asp Ile

710                                              720
Thr Ile Gln Gly Gly Asp Asp Val Phe Lys Glu Asn Tyr Val Thr

730
Leu Leu Gly Thr Phe Asp Glu Cys Tyr Pro Thr Tyr Leu Tyr Gln

740                                              750
Lys Ile Asp Glu Ser Lys Leu Lys Ala Tyr Thr Arg Tyr Gln Leu

760
Arg Gly Tyr Ile Glu Asp Ser Gln Asp Leu Glu Ile Tyr Leu Ile

770                                              780
Arg Tyr Asn Ala Lys His Glu Thr Val Asn Val Pro Gly Thr Gly

790
Ser Leu Trp Pro Leu Ser Ala Gln Ser Pro Ile Gly Lys Cys Gly

800                                              810
Glu Pro Asn Arg Cys Ala Pro His Leu Glu Trp Asn Pro Asp Leu

                                        820
Asp Cys Ser Cys Arg Asp Gly Glu Lys Cys Ala His His Ser His

            830                                         840
His Phe Ser Leu Asp Ile Asp Val Gly Cys Thr Asp Leu Asn Glu

                                        850
Asp Leu Gly Val Trp Val Ile Phe Lys Ile Lys Thr Gln Asp Gly

            860                                         870
His Ala Arg Leu Gly Asn Leu Glu Phe Leu Glu Glu Lys Pro Leu

                                        880
Val Gly Glu Ala Leu Ala Arg Val Lys Arg Ala Glu Lys Lys Trp

            890                                         900
Arg Asp Lys Arg Glu Lys Leu Glu Trp Glu Thr Asn Ile Val Tyr

                                        910
Lys Glu Ala Lys Glu Ser Val Asp Ala Leu Phe Val Asn Ser Gln

            920                                         930
Tyr Asp Arg Leu Gln Ala Asp Thr Asn Ile Ala Met Ile His Ala

                                        940
Ala Asp Lys Arg Val His Ser Ile Arg Glu Ala Tyr Leu Pro Glu

            950                                         960
Leu Ser Val Ile Pro Gly Val Asn Ala Ala Ile Phe Glu Glu Leu

                                        970
Glu Gly Arg Ile Phe Thr Ala Phe Ser Leu Tyr Asp Ala Arg Asn

            980                                         990
Val Ile Lys Asn Gly Asp Phe Asn Asn Gly Leu Ser Cys Trp Asn

                                        1000
Val Lys Gly His Val Asp Val Glu Glu Gln Asn Asn His Arg Ser

            1010                                        1020
Val Leu Val Val Pro Glu Trp Glu Ala Glu Val Ser Gln Glu Val

                                        1030
Arg Val Cys Pro Gly Arg Gly Tyr Ile Leu Arg Val Thr Ala Tyr

            1040                                        1050
Lys Glu Gly Tyr Gly Glu Gly Cys Val Thr Ile His Glu Ile Glu

                                        1060
Asn Asn Thr Asp Glu Leu Lys Phe Ser Asn Cys Val Glu Glu Glu

            1070                                        1080
Val Tyr Pro Asn Asn Thr Val Thr Cys Asn Asp Tyr Thr Ala Thr

                                        1090
Gln Glu Glu Tyr Glu Gly Thr Tyr Thr Ser Arg Asn Arg Gly Tyr

```
                    1100                                                    1110
     Asp Gly Ala Tyr Glu Ser Asn Ser Ser Val Pro Ala Asp Tyr Ala


                                                   1120
     Ser Ala Tyr Glu Glu Lys Ala Tyr Thr Asp Gly Arg Arg Asp Asn


                    1130                                                    1140
     Pro Cys Glu Ser Asn Arg Gly Tyr Gly Asp Tyr Thr Pro Leu Pro


                                                   1150
     Ala Gly Tyr Val Thr Lys Glu Leu Glu Tyr Phe Pro Glu Thr Asp


                    1160                                                    1170
     Lys Val Trp Ile Glu Ile Gly Glu Thr Glu Gly Thr Phe Ile Val


                                                   1180
     Asp Ser Val Glu Leu Leu Leu Met Glu Glu,
```

ou renfermant une séquence d'amino-acide comprenant au moins 723 amino-acides à partir de l'atome d'azote terminal de cette séquence d'amino-acide.

2. Un fragment d'ADN selon la revendicàtion 1, dans laquelle la séquence de nucléotide comprend la séquence suivante:

```
     ATG GAT AAC AAT CCG AAC ATC AAT GAA TGC ATT CCT TAT AAT TGT

     TTA AGT AAC CCT GAA GTA GAA GTA TTA GGT GGA GAA AGA GTG GAA      242

     ACT GGT TAC ACC CCA ATC GAT ATT TCC TTG TCG CTA ACG CAA TTT

     CTT TTG AGT GAA TTT GTT CCC GGT GCT GGA TTT GTG TTA GGA CTA      332

     GTT GAT ATA ATA TGG GGA ATT TTT GGT CCC TCT CAA TGG GAC GCA

     TTT CTT GTA CAA ATT GAA CAG TTA ATT AAC CAA AGA ATA GAA GAA      422

     TTC GCT AGG AAC CAA GCC ATT TCT AGA TTA GAA GGA CTA AGC AAT

     CTT TAT CAA ATT TAC GCA GAA TCT TTT AGA GAG TGG GAA GCA GAT      512

     CCT ACT AAT CCA GCA TTA AGA GAA GAG ATG CGT ATT CAA TTC AAT

     GAC ATG AAC AGT GCC CTT ACA ACC GCT ATT CCT CTT TTT GCA GTT      602

     CAA AAT TAT CAA GTT CCT CTT TTA TCA GTA TAT GTT CAA GCT GCA

     AAT TTA CAT TTA TCA GTT TTG AGA GAT GTT TCA GTG TTT GGA CAA      692

     AGG TGG GGA TTT GAT GCC GCG ACT ATC AAT AGT CGT TAT AAT GAT

     TTA ACT AGG CTT ATT GGC AAC TAT ACA GAT TAT CGT GTG CGC TGG      782

     TAC AAT ACG GGA TTA GAG CGT GTA TGG GGA CCG GAT TCT AGA GAT
```

33

```
TGG GTA AGG TAT AAT CAA TTT AGA AGA GAG CTA ACA CTT ACT GTA     872

TTA GAT ATC CTT GCT CTA TTC TCA AAT TAT GAT AGT CGA AGG TAT

CCA ATT CGA ACA GTT TCC CAA TTA ACA AGA GAA ATT TAT ACG AAC     962

CCA GTA TTA GAA AAT TTT GAT GGT AGT TTT CGT GGA ATG GCT CAG

AGA ATA GAA CAG AAT ATT AGG CAA CCA CAT CTT ATG GAT ATC CTT    1052

AAT AGG ATA ACC ATT TAT ACT GAT GTG CAT AGA GGC TTT AAT TAT

TGG TCA GGG CAT CAA ATA ACA GCT TCT CCT GTA GGG TTT TCA GGA    1142

CCA GAA TTC GCA TTC CCT TTA TTT GGG AAT GCG GGG AAT GCA GCT

CCA CCC GTA CTT GTC TCA TTA ACT GGT TTG GGG ATT TTT AGA ACA    1232

TTA TCT TCA CCT TTA TAT AGA AGA ATT ATA CTT GGT TCA GGC CCA

AAT AAT CAG GAA CTG TTT GTC CTT GAT GGA ACG GAG TTT TCT TTT    1322

GCC TCC CTA ACG ACC AAC TTG CCT TCC ACT ATA TAT AGA CAA AGG

GGT ACA GTC GAT TCA CTA GAT GTA ATA CCG CCA CAG GAT AAT AGT    1412

GTA CCA CCT CGT GCG GGA TTT AGC CAT CGA TTG AGT CAT GTT ACA

ATG CTG AGC CAA GCA GCT GGA GCA GTT TAC ACC TTG AGA GCT CCA    1502

ACG TTT TCT TGG CAG CAT CGC AGT GCT GAA TTT AAT AAT ATA ATT

CCT TCA TCA CAA ATT ACA CAA ATA CCT TTA ACA AAA TCT ACT AAT    1592

CTT GGC TCT GGA ACT TCT GTC GTT AAA GGA CCA GGA TTT ACA GGA

GGA GAT ATT CTT CGA AGA ACT TCA CCT GGC CAG ATT TCA ACC TTA    1682

AGA GTA AAT ATT ACT GCA CCA TTA TCA CAA AGA TAT CGG GTA AGA

ATT CGC TAC GCT TCT ACT ACA AAT TTA CAA TTC CAT ACA TCA ATT    1772

GAC GGA AGA CCT ATT AAT CAG GGT AAT TTT TCA GCA ACT ATG AGT

AGT GGG AGT AAT TTA CAG TCC GGA AGC TTT AGG ACT GTA GGT TTT    1862

ACT ACT CCG TTT AAC TTT TCA AAT GGA TCA AGT GTA TTT ACG TTA

AGT GCT CAT GTC TTC AAT TCA GGC AAT GAA GTT TAT ATA GAT CGA    1952

ATT GAA TTT GTT CCG GCA GAA GTA ACC TTT GAG GCA GAA TAT GAT

TTA GAA AGA GCA CAA AAG GCG GTG AAT GAG CTG TTT ACT TCT TCC    2042

AAT CAA ATC GGG TTA AAA ACA GAT GTG ACG GAT TAT CAT ATT GAT

CAA GTA TCC AAT TTA GTT GAG TGT TTA TCA GAT GAA TTT TGT CTG    2132
```

34

```
GAT GAA AAA CAA GAA TTG TCC GAG AAA GTC AAA CAT GCG AAG CGA

CTT AGT GAT GAG CGG AAT TTA CTT CAA GAT CCA AAC TTC AGA GGG      2222

ATC AAT AGA CAA CTA GAC CGT GGC TGG AGA GGA AGT ACG GAT ATT

ACC ATC CAA GGA GGC GAT GAC GTA TTC AAA GAG AAT TAC GTT ACG      2312

CTA TTG GGT ACC TTT GAT GAG TGC TAT CCA ACG TAT TTA TAT CAA

AAA ATA GAT GAG TCG AAA TTA AAA GCC TAT ACC CGT TAT CAA TTA      2402

AGA GGG TAT ATC GAA GAT AGT CAA GAC TTA GAA ATC TAT TTA ATT

CGC TAC AAT GCA AAA CAT GAA ACA GTA AAT GTG CCA GGT ACG GGT      2492

TCC TTA TGG CCG CTT TCA GCC CAA AGT CCA ATC GGA AAG TGT GGA

GAG CCG AAT CGA TGC GCG CCA CAC CTT GAA TGG AAT CCT GAC TTA      2582

GAT TGT TCG TGT AGG GAT GGA GAA AAA TGT GCC CAT CAT TCC CAT

CAT TTC TCC TTG GAC ATT GAT GTT GGA TGT ACA GAC TTA AAT GAG      2672

GAC TTA GGT GTA TGG GTG ATA TTC AAG ATT AAG ACG CAA GAT GGC

CAT GCA AGA CTA GGA AAT CTA GAA TTT CTC GAA GAG AAA CCA TTA      2762

GTA GGA GAA GCA CTA GCT CGT GTG AAA AGA GCC GAG AAA AAA TGG

AGA GAC AAA CGT GAA AAA TTG GAA TGG GAA ACA AAT ATT GTT TAT      2852

AAA GAG GCA AAA GAA TCT GTA GAT GCT TTA TTT GTA AAC TCT CAA

TAT GAT AGA TTA CAA GCG GAT ACC AAC ATC GCG ATG ATT CAT GCG      2942

GCA GAT AAA CGC GTT CAT AGC ATT CGA GAA GCT TAT CTG CCT GAG

CTG TCT GTG ATT CCG GGT GTC AAT GCG GCT ATT TTT GAA GAA TTA      3032

GAA GGG CGT ATT TTC ACT GCA TTC TCC CTA TAT GAT CGC AGA AAT

GTC ATT AAA AAT GGT GAT TTT AAT AAT GGC TTA TCC TGC TGG AAC      3122

GTG AAA GGG CAT GTA GAT GTA GAA GAA CAA AAC AAC CAC CGT TCG

GTC CTT GTT GTT CCG GAA TGG GAA GCA GAA GTG TCA CAA GAA GTT      3212

CGT GTC TGT CCG GGT CGT GGC TAT ATC CTT CGT GTC ACA GCG TAC

AAG GAG GGA TAT GGA GAA GGT TGC GTA ACC ATT CAT GAG ATC GAG      3302

AAC AAT ACA GAC GAA CTG AAG TTT AGC AAC TGT GTA GAA GAG GAA

GTA TAT CCA AAC AAC ACG GTA ACG TGT AAT GAT TAT ACT GCG ACT      3392

CAA GAA GAA TAT GAG GGT ACG TAC ACT TCT CGT AAT CGA GGA TAT
```

35

```
GAC GGA GCC TAT GAA AGC AAT TCT TCT GTA CCA GCT GAT TAT GCA      3482

TCA GCC TAT GAA GAA AAA GCA TAT ACA GAT GGA CGA AGA GAC AAT

CCT. TGT GAA TCT AAC AGA GGA TAT GGG GAT TAC ACA CCA CTA CCA      3572

GCT GGC TAT GTG ACA AAA GAA TTA GAG TAC TTC CCA GAA ACC GAT

AAG GTA TGG ATT GAG ATC GGA GAA ACG GAA GGA ACA TTC ATC GTG      3662

GAC AGC GTG GAA TTA CTT CTT ATG GAG GAA,
```

ou une séquence comprenant au moins 723 codons à partir du terminus-5' de cette séquence de nucléotide.

3. Un fragment d'ADN selon la revendication 1 ou la revendication 2, d'une longueur d'environ 6,6 kb.

4. Un fragment d'ADN selon la revendication 1, caractérisé en ce que la séquence d'amino-acide encodé par la séquence de nucléotide comprend une séquence d'amino-acide débutant au motif Met à la position amino-acide 1 et se terminant au motif Arg à la position amino-acide 934, telle que définie dans la revendication 1.

5. Un fragment d'ADN selon la revendication 4, caractérise en ce que la séquence de nucléotide codant pour la séquence d'amino-acide comprend une séquence de nucléotide débutant à partir de la position 153 et se terminant en position 2955, telle que définie dans la revendication 2.

6. Un fragment d'ADN selon l'une quelconque des revendications 1, 4 et 5, d'une longueur d'environ 2,95 kb.

7. Un plasmide capable de se répliquer dans E. coli et d'exprimer le peptide défini dans la revendication 1, comprenant un plasmide pBR322 dans lequel on a inséré la séquence d'ADN définie dans la revendication 1.

8. Un plasmide selon la revendication 7, caractérisé en ce que la longueur du fragment d'ADN inséré est égale à environ 6,6 kb.

9. Un plasmide selon la revendication 8, caractérisé en ce que le fragment d'ADN a été inséré au site de clivage PstI dans le plasmide pBR322.

10. Un plasmide selon la revendication 7, caractérisé en ce que la longueur du fragment d'ADN inséré est égale à environ 2,95 kb.

11. Un plasmide selon la revendication 10, caractérisé en ce que le fragment d'ADN a été inséré au site de clivage BamHI dans le plasmide pBR322.

12. E. coli transformé à l'aide d'un plasmide selon l'une quelconque des revendications 7 à 11 et sécrétant une protéine insecticide.

13. E. coli selon la revendication 12, caractérisé en ce que le poids moléculaire de la protéine est égal à 144 kD.

14. E. coli selon la revendication 12, caractérisé en ce que le poids moléculaire de la protéine est égal à 75 kD.

15. Une protéine insecticide obtenue à partir d'un organisme transformé de façon à incorporer un fragment d'ADN selon l'une quelconque des revendications 1 à 6.

16. Une composition comprenant une quantité insecticide efficace de protéine selon la revendication 15, en même temps qu'un excipient.

17. Un procédé de préparation d'une protéine insecticide comprenant la production de cette protéine à partir d'un organisme transformé de façon à incorporer un fragment d'ADN correspondant à l'une quelconque des revendications 1 à 6.

REVENDICATIONS

pour l'etat contractant: AT

1. Un procédé consistant à préparer un fragment d'ADN comprenant une séquence de nucléotide codant pour un peptide insecticide renfermant la séquence suivante d'amino-acide:

```
1                                              10
Met Asp Asn Asn Pro Asn Ile Asn Glu Cys Ile Pro Tyr Asn Cys

                    20                                    30
Leu Ser Asn Pro Glu Val Glu Val Leu Gly Gly Glu Arg Ile Glu

                              40
Thr Gly Tyr Thr Pro Ile Asp Ile Ser Leu Ser Leu Thr Gln Phe

              50                                    60
Leu Leu Ser Glu Phe Val Pro Gly Ala Gly Phe Val Leu Gly Leu

                                    70
Val Asp Ile Ile Trp Gly Ile Phe Gly Pro Ser Gln Trp Asp Ala

                    80                                    90
Phe Leu Val Gln Ile Glu Gln Leu Ile Asn Gln Arg Ile Glu Glu

                                    100
Phe Ala Arg Asn Gln Ala Ile Ser Arg Leu Glu Gly Leu Ser Asn

                    110                                    120
Leu Tyr Gln Ile Tyr Ala Glu Ser Phe Arg Glu Trp Glu Ala Asp

                                    130
Pro Thr Asn Pro Ala Leu Arg Glu Glu Met Arg Ile Gln Phe Asn

                    140                                    150
Asp Met Asn Ser Ala Leu Thr Thr Ala Ile Pro Leu Phe Ala Val

                                    160
Gln Asn Tyr Gln Val Pro Leu Leu Ser Val Tyr Val Gln Ala Ala

                    170                                    180
Asn Leu His Leu Ser Val Leu Arg Asp Val Ser Val Phe Gly Gln

                                    190
Arg Trp Gly Phe Asp Ala Ala Thr Ile Asn Ser Arg Tyr Asn Asp

              200                                    210
Leu Thr Arg Leu Ile Gly Asn Tyr Thr Asp Tyr Ala Val Arg Trp

                                    220
Tyr Asn Thr Gly Leu Glu Arg Val Trp Gly Pro Asp Ser Arg Asp

                    230                                    240
Trp Val Arg Try Asn Gln Phe Arg Arg Glu Leu Thr Leu Thr Val
```

                                                    250
Leu Asp Ile Val Ala Leu Phe Ser Asn Tyr Asp Ser Arg Arg Tyr

                    260                                     270
Pro Ile Arg Thr Val Ser Gln Leu Thr Arg Glu Ile Tyr Thr Asn

                                        280
Pro Val Leu Glu Asn Phe Asp Gly Ser Phe Arg Gly Met Ala Gln

                    290                                     300
Arg Ile Glu Gln Asn Ile Arg Gln Pro His Leu Met Asp Ile Leu

                                        310
Asn Arg Ile Thr Ile Tyr Thr Asp Val His Arg Gly Phe Asn Tyr

                    320                                     330
Trp Ser Gly His Gln Ile Thr Ala Ser Pro Val Gly Phe Ser Gly

                                        340
Pro Glu Phe Ala Phe Pro Leu Phe Gly Asn Ala Gly Asn Ala Ala

                    350                                     360
Pro Pro Val Leu Val Ser Leu Thr Gly Leu Gly Ile Phe Arg Thr

                                        370
Leu Ser Ser Pro Leu Tyr Arg Arg Ile Ile Leu Gly Ser Gly Pro

                    380                                     390
Asn Asn Gln Glu Leu Phe Val Leu Asp Gly Thr Glu Phe Ser Phe

                                        400
Ala Ser Leu Thr Thr Asn Leu Pro Ser Thr Ile Tyr Arg Gln Arg

                    410                                     420
Gly Thr Val Asp Ser Leu Asp Val Ile Pro Pro Gln Asp Asn Ser

                                        430
Val Pro Pro Arg Ala Gly Phe Ser His Arg Leu Ser His Val Thr

                    440                                     450
Met Leu Ser Gln Ala Ala Gly Ala Val Tyr Thr Leu Arg Ala Pro

                                        460
Thr Phe Ser Trp Gln His Arg Ser Ala Glu Phe Asn Asn Ile Ile

                    470                                     480
Pro Ser Ser Gln Ile Thr Gln Ile Pro Leu Thr Lys Ser Thr Asn

                                        490
Leu Gly Ser Gly Thr Ser Val Val Lys Gly Pro Gly Phe Thr Gly

                    500                                     510
Gly Asp Ile Leu Arg Arg Thr Ser Pro Gly Gln Ile Ser Thr Leu

                                        520
Arg Val Asn Ile Thr Ala Pro Leu Ser Gln Arg Tyr Arg Val Arg

```
                    530                                             540
        Ile Arg Tyr Ala Ser Thr Thr Asn Leu Gln Phe His Thr Ser Ile

                                                550
        Asp Gly Arg Pro Ile Asn Gln Gly Asn Phe Ser Ala Thr Met Ser

                    560                                             570
        Ser Gly Ser Asn Leu Gln Ser Gly Ser Phe Arg Thr Val Gly Phe

                                        580
        Thr Thr Pro Phe Asn Phe Ser Asn Gly Ser Ser Val Phe Thr Leu

                    590                                             600
        Ser Ala His Val Phe Asn Ser Gly Asn Glu Val Tyr Ile Asp Arg

                                            610
        Ile Glu Phe Val Pro Ala Glu Val Thr Phe Glu Ala Glu Tyr Asp

                    620                                             630
        Leu Glu Arg Ala Gln Lys Ala Val Asn Glu Leu Phe Thr Ser Ser

                                        640
        Asn Gln Ile Gly Leu Lys Thr Asp Val Thr Asp Tyr His Ile Asp

                    650                                             660
        Gln Val Ser Asn Leu Val Glu Cys Leu Ser Asp Glu Phe Cys Leu

                                        670
        Asp Glu Lys Gln Glu Leu Ser Glu Lys Val Lys His Ala Lys Arg

                    680                                             690
        Leu Ser Asp Glu Arg Asn Leu Leu Gln Asp Pro Asn Phe Arg Gly

                                            700
        Ile Asn Arg Gln Leu Asp Arg Gly Trp Arg Gly Ser Thr Asp Ile

                    710                                             720
        Thr Ile Gln Gly Gly Asp Asp Val Phe Lys Glu Asn Tyr Val Thr

                                        730
        Leu Leu Gly Thr Phe Asp Glu Cys Tyr Pro Thr Tyr Leu Tyr Gln

                    740                                             750
        Lys Ile Asp Glu Ser Lys Leu Lys Ala Tyr Thr Arg Tyr Gln Leu

                                            760
        Arg Gly Tyr Ile Glu Asp Ser Gln Asp Leu Glu Ile Tyr Leu Ile

                    770                                             780
        Arg Tyr Asn Ala Lys His Glu Thr Val Asn Val Pro Gly Thr Gly

                                        790
        Ser Leu Trp Pro Leu Ser Ala Gln Ser Pro Ile Gly Lys Cys Gly

                    800                                             810
        Glu Pro Asn Arg Cys Ala Pro His Leu Glu Trp Asn Pro Asp Leu
```

                                    820
Asp Cys Ser Cys Arg Asp Gly Glu Lys Cys Ala His His Ser His

        830                                             840
His Phe Ser Leu Asp Ile Asp Val Gly Cys Thr Asp Leu Asn Glu

                                    850
Asp Leu Gly Val Trp Val Ile Phe Lys Ile Lys Thr Gln Asp Gly

                    860                             870
His Ala Arg Leu Gly Asn Leu Glu Phe Leu Glu Glu Lys Pro Leu

                                    880
Val Gly Glu Ala Leu Ala Arg Val Lys Arg Ala Glu Lys Lys Trp

            890                                     900
Arg Asp Lys Arg Glu Lys Leu Glu Trp Glu Thr Asn Ile Val Tyr

                                    910
Lys Glu Ala Lys Glu Ser Val Asp Ala Leu Phe Val Asn Ser Gln

            920                                     930
Tyr Asp Arg Leu Gln Ala Asp Thr Asn Ile Ala Met Ile His Ala

                                    940
Ala Asp Lys Arg Val His Ser Ile Arg Glu Ala Tyr Leu Pro Glu

            950                                     960
Leu Ser Val Ile Pro Gly Val Asn Ala Ala Ile Phe Glu Glu Leu

                                    970
Glu Gly Arg Ile Phe Thr Ala Phe Ser Leu Tyr Asp Ala Arg Asn

            980                                     990
Val Ile Lys Asn Gly Asp Phe Asn Asn Gly Leu Ser Cys Trp Asn

                                    1000
Val Lys Gly His Val Asp Val Glu Glu Gln Asn Asn His Arg Ser

            1010                                    1020
Val Leu Val Val Pro Glu Trp Glu Ala Glu Val Ser Gln Glu Val

                                    1030
Arg Val Cys Pro Gly Arg Gly Tyr Ile Leu Arg Val Thr Ala Tyr

            1040                                    1050
Lys Glu Gly Tyr Gly Glu Gly Cys Val Thr Ile His Glu Ile Glu

                                    1060
Asn Asn Thr Asp Glu Leu Lys Phe Ser Asn Cys Val Glu Glu Glu

            1070                                    1080
Val Tyr Pro Asn Asn Thr Val Thr Cys Asn Asp Tyr Thr Ala Thr

                                    1090
Gln Glu Glu Tyr Glu Gly Thr Tyr Thr Ser Arg Asn Arg Gly Tyr

```
                    1100                                          1110
    Asp Gly Ala Tyr Glu Ser Asn Ser Ser Val Pro Ala Asp Tyr Ala

                                             1120
    Ser Ala Tyr Glu Glu Lys Ala Tyr Thr Asp Gly Arg Arg Asp Asn

                    1130                                          1140
    Pro Cys Glu Ser Asn Arg Gly Tyr Gly Asp Tyr Thr Pro Leu Pro

                                             1150
    Ala Gly Tyr Val Thr Lys Glu Leu Glu Tyr Phe Pro Glu Thr Asp

                    1160                                          1170
    Lys Val Trp Ile Glu Ile Gly Glu Thr Glu Gly Thr Phe Ile Val

                                             1180
    Asp Ser Val Glu Leu Leu Leu Met Glu Glu,
```

ou renfermant une séquence d'amino-acide comprenant au moins 723 amino-acides à partir de l'atome d'azote terminal de cette séquence d'amino-acide.

2. Un procédé selon la revendication 1, caractérisé en ce que la séquence de nucléotide comprend la séquence suivante:

```
    ATG GAT AAC AAT CCG AAC ATC AAT GAA TGC ATT CCT TAT AAT TGT

    TTA AGT AAC CCT GAA GTA GAA GTA TTA GGT GGA GAA AGA GTG GAA      242

    ACT GGT TAC ACC CCA ATC GAT ATT TCC TTG TCG CTA ACG CAA TTT

    CTT TTG AGT GAA TTT GTT CCC GGT GCT GGA TTT GTG TTA GGA CTA      332

    GTT GAT ATA ATA TGG GGA ATT TTT GGT CCC TCT CAA TGG GAC GCA

    TTT CTT GTA CAA ATT GAA CAG TTA ATT AAC CAA AGA ATA GAA GAA      422

    TTC GCT AGG AAC CAA GCC ATT TCT AGA TTA GAA GGA CTA AGC AAT

    CTT TAT CAA ATT TAC GCA GAA TCT TTT AGA GAG TGG GAA GCA GAT      512

    CCT ACT AAT CCA GCA TTA AGA GAA GAG ATG CGT ATT CAA TTC AAT

    GAC ATG AAC AGT GCC CTT ACA ACC GCT ATT CCT CTT TTT GCA GTT      602

    CAA AAT TAT CAA GTT CCT CTT TTA TCA GTA TAT GTT CAA GCT GCA

    AAT TTA CAT TTA TCA GTT TTG AGA GAT GTT TCA GTG TTT GGA CAA      692

    AGG TGG GGA TTT GAT GCC GCG ACT ATC AAT AGT CGT TAT AAT GAT

    TTA ACT AGG CTT ATT GGC AAC TAT ACA GAT TAT CGT GTG CGC TGG      782

    TAC AAT ACG GGA TTA GAG CGT GTA TGG GGA CCG GAT TCT AGA GAT
```

```
TGG GTA AGG TAT AAT CAA TTT AGA AGA GAG CTA ACA CTT ACT GTA        872

TTA GAT ATC CTT GCT CTA TTC TCA AAT TAT GAT AGT CGA AGG TAT

CCA ATT CGA ACA GTT TCC CAA TTA ACA AGA GAA ATT TAT ACG AAC        962

CCA GTA TTA GAA AAT TTT GAT GGT AGT TTT CGT GGA ATG GCT CAG

AGA ATA GAA CAG AAT ATT AGG CAA CCA CAT CTT ATG GAT ATC CTT       1052

AAT AGG ATA ACC ATT TAT ACT GAT GTG CAT AGA GGC TTT AAT TAT

TGG TCA GGG CAT CAA ATA ACA GCT TCT CCT GTA GGG TTT TCA GGA       1142

CCA GAA TTC GCA TTC CCT TTA TTT GGG AAT GCG GGG AAT GCA GCT

CCA CCC GTA CTT GTC TCA TTA ACT GGT TTG GGG ATT TTT AGA ACA       1232

TTA TCT TCA CCT TTA TAT AGA AGA ATT ATA CTT GGT TCA GGC CCA

AAT AAT CAG GAA CTG TTT GTC CTT GAT GGA ACG GAG TTT TCT TTT       1322

GCC TCC CTA ACG ACC AAC TTG CCT TCC ACT ATA TAT AGA CAA AGG

GGT ACA GTC GAT TCA CTA GAT GTA ATA CCG CCA CAG GAT AAT AGT       1412

GTA CCA CCT CGT GCG GGA TTT AGC CAT CGA TTG AGT CAT GTT ACA

ATG CTG AGC CAA GCA GCT GGA GCA GTT TAC ACC TTG AGA GCT CCA       1502

ACG TTT TCT TGG CAG CAT CGC AGT GCT GAA TTT AAT AAT ATA ATT

CCT TCA TCA CAA ATT ACA CAA ATA CCT TTA ACA AAA TCT ACT AAT       1592

CTT GGC TCT GGA ACT TCT GTC GTT AAA GGA CCA GGA TTT ACA GGA

GGA GAT ATT CTT CGA AGA ACT TCA CCT GGC CAG ATT TCA ACC TTA       1682

AGA GTA AAT ATT ACT GCA CCA TTA TCA CAA AGA TAT CGG GTA AGA

ATT CGC TAC GCT TCT ACT ACA AAT TTA CAA TTC CAT ACA TCA ATT       1772

GAC GGA AGA CCT ATT AAT CAG GGT AAT TTT TCA GCA ACT ATG AGT

AGT GGG AGT AAT TTA CAG TCC GGA AGC TTT AGG ACT GTA GGT TTT       1862

ACT ACT CCG TTT AAC TTT TCA AAT GGA TCA AGT GTA TTT ACG TTA

AGT GCT CAT GTC TTC AAT TCA GGC AAT GAA GTT TAT ATA GAT CGA       1952

ATT GAA TTT GTT CCG GCA GAA GTA ACC TTT GAG GCA GAA TAT GAT

TTA GAA AGA GCA CAA AAG GCG GTG AAT GAG CTG TTT ACT TCT TCC       2042

AAT CAA ATC GGG TTA AAA ACA GAT GTG ACG GAT TAT CAT ATT GAT

CAA GTA TCC AAT TTA GTT GAG TGT TTA TCA GAT GAA TTT TGT CTG       2132
```

```
GAT GAA AAA CAA GAA TTG TCC GAG AAA GTC AAA CAT GCG AAG CGA

CTT AGT GAT GAG CGG AAT TTA CTT CAA GAT CCA AAC TTC AGA GGG      2222

ATC AAT AGA CAA CTA GAC CGT GGC TGG AGA GGA AGT ACG GAT ATT

ACC ATC CAA GGA GGC GAT GAC GTA TTC AAA GAG AAT TAC GTT ACG      2312

CTA TTG GGT ACC TTT GAT GAG TGC TAT CCA ACG TAT TTA TAT CAA

AAA ATA GAT GAG TCG AAA TTA AAA GCC TAT ACC CGT TAT CAA TTA      2402

AGA GGG TAT ATC GAA GAT AGT CAA GAC TTA GAA ATC TAT TTA ATT

CGC TAC AAT GCA AAA CAT GAA ACA GTA AAT GTG CCA GGT ACG GGT      2492

TCC TTA TGG CCG CTT TCA GCC CAA AGT CCA ATC GGA AAG TGT GGA

GAG CCG AAT CGA TGC GCG CCA CAC CTT GAA TGG AAT CCT GAC TTA      2582

GAT TGT TCG TGT AGG GAT GGA GAA AAA TGT GCC CAT CAT TCC CAT

CAT TTC TCC TTG GAC ATT GAT GTT GGA TGT ACA GAC TTA AAT GAG      2672

GAC TTA GGT GTA TGG GTG ATA TTC AAG ATT AAG ACG CAA GAT GGC

CAT GCA AGA CTA GGA AAT CTA GAA TTT CTC GAA GAG AAA CCA TTA      2762

GTA GGA GAA GCA CTA GCT CGT GTG AAA AGA GCC GAG AAA AAA TGG

AGA GAC AAA CGT GAA AAA TTG GAA TGG GAA ACA AAT ATT GTT TAT      2852

AAA GAG GCA AAA GAA TCT GTA GAT GCT TTA TTT GTA AAC TCT CAA

TAT GAT AGA TTA CAA GCG GAT ACC AAC ATC GCG ATG ATT CAT GCG      2942

GCA GAT AAA CGC GTT CAT AGC ATT CGA GAA GCT TAT CTG CCT GAG

CTG TCT GTG ATT CCG GGT GTC AAT GCG GCT ATT TTT GAA GAA TTA      3032

GAA GGG CGT ATT TTC ACT GCA TTC TCC CTA TAT GAT CGC AGA AAT

GTC ATT AAA AAT GGT GAT TTT AAT AAT GGC TTA TCC TGC TGG AAC      3122

GTG AAA GGG CAT GTA GAT GTA GAA GAA CAA AAC AAC CAC CGT TCG

GTC CTT GTT GTT CCG GAA TGG GAA GCA GAA GTG TCA CAA GAA GTT      3212

CGT GTC TGT CCG GGT CGT GGC TAT ATC CTT CGT GTC ACA GCG TAC

AAG GAG GGA TAT GGA GAA GGT TGC GTA ACC ATT CAT GAG ATC GAG      3302

AAC AAT ACA GAC GAA CTG AAG TTT AGC AAC TGT GTA GAA GAG GAA

GTA TAT CCA AAC AAC ACG GTA ACG TGT AAT GAT TAT ACT GCG ACT      3392

CAA GAA GAA TAT GAG GGT ACG TAC ACT TCT CGT AAT CGA GGA TAT
```

44

```
GAC GGA GCC TAT GAA AGC AAT TCT TCT GTA CCA GCT GAT TAT GCA    3482

TCA GCC TAT GAA GAA AAA GCA TAT ACA GAT GGA CGA AGA GAC AAT

CCT TGT GAA TCT AAC AGA GGA TAT GGG GAT TAC ACA CCA CTA CCA    3572

GCT GGC TAT GTG ACA AAA GAA TTA GAG TAC TTC CCA GAA ACC GAT

AAG GTA TGG ATT GAG ATC GGA GAA ACG GAA GGA ACA TTC ATC GTG    3662

GAC AGC GTG GAA TTA CTT CTT ATG GAG GAA.
```

ou une séquence comprenant au moins 723 codons à partir du terminus-5' de cette séquence de nucléotide.

3. Un procédé selon la revendication 1 ou la revendication 2, caractérisé en ce que la longueur du fragment d'ADN est égal à environ 6,6 kb.

4. Un procédé selon la revendication 1, caractérisé en ce que la séquence d'amino-acide encodée par la séquence de nucléotide comprend une séquence d'amino-acide débutant au motif Met à la position amino-acide 1 et se terminant au motif Arg à la position amino-acide 934, telle que définie dans la revendication 1.

5. Un procédé selon la revendication 4, caractérisé en ce que la séquence de nucléotide codant pour la séquence d'amino-acide comprend une séquence de nucléotide débutant à partir de la position 153 et se terminant en position 2955, telle que définie dans la revendication 2.

6. Un procédé selon l'une quelconque des revendications 1, 4 et 5, caractérisé en ce que la longueur du fragment d'ADN est égal à environ 2,95 kb.

7. Un procédé consistant à préparer un plasmide capable de se répliquer dans E. coli et d'exprimer le peptide défini dans la revendication 1, consistant à insérer une séquence d'ADN telle que définie dans la revendication 1 dans un plasmide pBR322.

8. Un procédé selon la revendication 7, caractérisé en ce que la longueur du fragment d'ADN inséré est égale à environ 6,6 kb.

9. Un procédé selon la revendication 8, dans lequel le fragment d'ADN est inséré au site de clivage PstI dans le plasmide pBR322.

10. Un procédé selon la revendication 7, caractérisé en ce que la longueur du fragment d'ADN inséré est égale à environ 2,95 kb.

11. Un procédé selon la revendication 10, caractérisé en ce que le fragment d'ADN est inséré au site de clivage BamHI dans le plasmide pBR322.

12. E. coli transformé à l'aide d'un plasmide préparé selon l'une quelconque des revendications 7 à 11 et sécrétant une protéine insecticide.

13. E. coli selon la revendication 12, caractérisé en ce que le poids moléculaire de la protéine est égal à 144 kD.

14. E. coli selon la revendication 12, caractérisé en ce que le poids moléculaire de la protéine est égal à 75 kD.

15. Un procédé de préparation d'une protéine insecticide consistant à préparer cette protéine à partir d'un organisme transformé de façon à incorporer un fragment d'ADN selon l'une quelconque des revendications 1 à '6.

16. Un Procédé selon la revendication 15, caractérisé en ce que cet organisme est le E. coli selon l'une quelconque des revendications 12, 13 et 14.


**Ansprüche**

1. DNA-Fragment, enthaltend eine für ein insektizides Peptid codierende Nucleotidsequenz mit der folgenden Aminosäuresequenz:

```
     1                                      10
    Met Asp Asn Asn Pro Asn Ile Asn Glu Cys Ile Pro Tyr Asn Cys

                         20                                      30
    Leu Ser Asn Pro Glu Val Glu Val Leu Gly Gly Glu Arg Ile Glu

                                             40
    Thr Gly Tyr Thr Pro Ile Asp Ile Ser Leu Ser Leu Thr Gln Phe

                         50                                      60
    Leu Leu Ser Glu Phe Val Pro Gly Ala Gly Phe Val Leu Gly Leu

                                             70
    Val Asp Ile Ile Trp Gly Ile Phe Gly Pro Ser Gln Trp Asp Ala

                         80                                      90
    Phe Leu Val Gln Ile Glu Gln Leu Ile Asn Gln Arg Ile Glu Glu

                                            100
    Phe Ala Arg Asn Gln Ala Ile Ser Arg Leu Glu Gly Leu Ser Asn

                        110                                     120
    Leu Tyr Gln Ile Tyr Ala Glu Ser Phe Arg Glu Trp Glu Ala Asp

                                            130
    Pro Thr Asn Pro Ala Leu Arg Glu Glu Met Arg Ile Gln Phe Asn

                        140                                     150
    Asp Met Asn Ser Ala Leu Thr Thr Ala Ile Pro Leu Phe Ala Val

                                            160
    Gln Asn Tyr Gln Val Pro Leu Leu Ser Val Tyr Val Gln Ala Ala

                        170                                     180
    Asn Leu His Leu Ser Val Leu Arg Asp Val Ser Val Phe Gly Gln

                                            190
    Arg Trp Gly Phe Asp Ala Ala Thr Ile Asn Ser Arg Tyr Asn Asp

                        200                                     210
    Leu Thr Arg Leu Ile Gly Asn Tyr Thr Asp Tyr Ala Val Arg Trp
```

220
Tyr Asn Thr Gly Leu Glu Arg Val Trp Gly Pro Asp Ser Arg Asp

230                                                240
Trp Val Arg Try Asn Gln Phe Arg Arg Glu Leu Thr Leu Thr Val

250
Leu Asp Ile Val Ala Leu Phe Ser Asn Tyr Asp Ser Arg Arg Tyr

260                                                270
Pro Ile Arg Thr Val Ser Gln Leu Thr Arg Glu Ile Tyr Thr Asn

280
Pro Val leu Glu Asn Phe Asp Gly Ser Phe Arg Gly Met Ala Gln

290                                                300
Arg Ile Glu Gln Asn Ile Arg Gln Pro His Leu Met Asp Ile Leu

310
Asn Arg Ile Thr Ile Tyr Thr Asp Val His Arg Gly Phe Asn Tyr

320                                                330
Trp Ser Gly His Gln Ile Thr Ala Ser Pro Val Gly Phe Ser Gly

340
Pro Glu Phe Ala Phe Pro Leu Phe Gly Asn Ala Gly Asn Ala Ala

350                                                360
Pro Pro Val Leu Val Ser Leu Thr Gly Leu Gly Ile Phe Arg Thr

370
Leu Ser Ser Pro Leu Tyr Arg Arg Ile Ile Leu Gly Ser Gly Pro

380                                                390
Asn Asn Gln Glu Leu Phe Val Leu Asp Gly Thr Glu Phe Ser Phe

400
Ala Ser Leu Thr Thr Asn Leu Pro Ser Thr Ile Tyr Arg Gln Arg

410                                                420
Gly Thr Val Asp Ser Leu Asp Val Ile Pro Pro Gln Asp Asn Ser

430
Val Pro Pro Arg Ala Gly Phe Ser His Arg Leu Ser His Val Thr

440                                                450
Met Leu Ser Gln Ala Ala Gly Ala Val Tyr Thr Leu Arg Ala Pro

460
Thr Phe Ser Trp Gln His Arg Ser Ala Glu Phe Asn Asn Ile Ile

470                                                480
Pro Ser Ser Gln Ile Thr Gln Ile Pro Leu Thr Lys Ser Thr Asn

```
                                                          490
Leu Gly Ser Gly Thr Ser Val Val Lys Gly Pro Gly Phe Thr Gly

         500                                         510
Gly Asp Ile Leu Arg Arg Thr Ser Pro Gly Gln Ile Ser Thr Leu

                                    520
Arg Val Asn Ile Thr Ala Pro Leu Ser Gln Arg Tyr Arg Val Arg

         530                                         540
Ile Arg Tyr Ala Ser Thr Thr Asn Leu Gln Phe His Thr Ser Ile

                                    550
Asp Gly Arg Pro Ile Asn Gln Gly Asn Phe Ser Ala Thr Met Ser

         560                                         570
Ser Gly Ser Asn Leu Gln Ser Gly Ser Phe Arg Thr Val Gly Phe

                                    580
Thr Thr Pro Phe Asn Phe Ser Asn Gly Ser Ser Val Phe Thr Leu

         590                                         600
Ser Ala His Val Phe Asn Ser Gly Asn Glu Val Tyr Ile Asp Arg

                                    610
Ile Glu Phe Val Pro Ala Glu Val Thr Phe Glu Ala Glu Tyr Asp

         620                                         630
Leu Glu Arg Ala Gln Lys Ala Val Asn Glu Leu Phe Thr Ser Ser

                                    640
Asn Gln Ile Gly Leu Lys Thr Asp Val Thr Asp Tyr His Ile Asp

         650                                         660
Gln Val Ser Asn Leu Val Glu Cys Leu Ser Asp Glu Phe Cys Leu

                                    670
Asp Glu Lys Gln Glu Leu Ser Glu Lys Val Lys His Ala Lys Arg

         680                                         690
Leu Ser Asp Glu Arg Asn Leu Leu Gln Asp Pro Asn Phe Arg Gly

                                    700
Ile Asn Arg Gln Leu Asp Arg Gly Trp Arg Gly Ser Thr Asp Ile

         710                                         720
Thr Ile Gln Gly Gly Asp Asp Val Phe Lys Glu Asn Tyr Val Thr

                                    730
Leu Leu Gly Thr Phe Asp Glu Cys Tyr Pro Thr Tyr Leu Tyr Gln

                                                     750
Lys Ile Asp Glu Ser Lys Leu Lys Ala Tyr Thr Arg Tyr Gln Leu
```

760
Arg Gly Tyr Ile Glu Asp Ser Gln Asp Leu Gln Ile Tyr Leu Ile

770                                         780
Arg Tyr Asn Ala Lys His Glu Thr Val Asn Val Pro Gly Thr Gly

790
Ser Leu Trp Pro Leu Ser Ala Gln Ser Pro Ile Gly Lys Cys Gly

800                                         810
Glu Pro Asn Arg Cys Ala Pro His Leu Glu Trp Asn Pro Asp Leu

820
Asp Cys Ser Cys Arg Asp Gly Glu Lys Cys Ala His His Ser His

830                                         840
His Phe Ser Leu Asp Ile Asp Val Gly Cys Thr Asp Leu Asn Glu

850
Asp Leu Gly Val Trp Val Ile Phe Lys Ile Lys Thr Gln Asp Gly

860                                         870
His Ala Arg Leu Gly Asn Leu Glu Phe Leu Glu Glu Lys Pro Leu

880
Val Gly Glu Ala Leu Ala Arg Val Lys Arg Ala Glu Lys Lys Trp

890                                         900
Arg Asp Lys Arg Glu Lys Leu Glu Trp Glu Thr Asn Ile Val Tyr

910
Lys Glu Ala Lys Glu Ser Val Asp Ala Leu Phe Val Asn Ser Gln

920                                         930
Tyr Asp Arg Leu Gln Ala Asp Thr Asn Ile Ala Met Ile His Ala

940
Ala Asp Lys Arg Val His Ser Ile Arg Glu Ala Tyr Leu Pro Glu

950                                         960
Leu Ser Val Ile Pro Gly Val Asn Ala Ala Ile Phe Glu Glu Leu

970
Glu Gly Arg Ile Phe Thr Ala Phe Ser Leu Tyr Asp Ala Arg Asn

980                                         990
Val Ile Lys Asn Gly Asp Phe Asn Asn Gly Leu Ser Cys Trp Asn

1000
Val Lys Gly His Val Asp Val Glu Glu Gln Asn Asn His Arg Ser

1010                                        1020
Val Leu Val Val Pro Glu Trp Glu Ala Glu Val Ser Gln Glu Val

```
                                      1030
       Arg Val Cys Pro Gly Arg Gly Tyr Ile Leu Arg Val Thr Ala Tyr

          1040                                    1050
       Lys Glu Gly Tyr Gly Glu Gly Cys Val Thr Ile His Glu Ile Glu

                                      1060
       Asn Asn Thr Asp Glu Leu Lys Phe Ser Asn Cys Val Glu Glu Glu

             1070                                    1080
       Val Tyr Pro Asn Asn Thr Val Thr Cys Asn Asp Tyr Thr Ala Thr

                                      1090
       Gln Glu Glu Tyr Glu Gly Thr Tyr Thr Ser Arg Asn Arg Gly Tyr

          1100                                    1110
       Asp Gly Ala Tyr Glu Ser Asn Ser Ser Val Pro Ala Asp Tyr Ala

                                      1120
       Ser Ala Tyr Glu Glu Lys Ala Tyr Thr Asp Gly Arg Arg Asp Asn

             1130                                    1140
       Pro Cys Glu Ser Asn Arg Gly Tyr Gly Asp Tyr Thr Pro Leu Pro

                                      1150
       Ala Gly Tyr Val Thr Lys Glu Leu Glu Tyr Phe Pro Glu Thr Asp

             1160                                    1170
       Lys Val Trp Ile Glu Ile Gly Glu Thr Glu Gly Thr Phe Ile Val

                                      1180
       Asp Ser Val Glu Leu Leu Leu Met Glu Glu,
```

oder eine Aminosäuresequenz, die mindestens 723 Aminosäuren von dem N-Endpunkt dieser Aminosäuresequenz aus enthält.

2. DNA-Fragment nach Anspruch 1, wobei die Nucleotidsequenz die folgende Sequenz enthält:

50

```
ATG GAT AAC AAT CCG AAC ATC AAT GAA TGC ATT CCT TAT AAT TGT
TTA AGT AAC CCT GAA GTA GAA GTA TTA GGT GGA GAA AGA GTG GAA  242
ACT GGT TAC ACC CCA ATC GAT ATT TCC TTG TCG CTA ACG CAA TTT
CTT TTG AGT GAA TTT GTT CCC GGT GCT GGA TTT GTG TTA GGA CTA  332
GTT GAT ATA ATA TGG GGA ATT TTT GGT CCC TCT CAA TGG GAC GCA
TTT CTT GTA CAA ATT GAA CAG TTA ATT AAC CAA AGA ATA GAA GAA  422
TTC GCT AGG AAC CAA GCC ATT TCT AGA TTA GAA GGA CTA AGC AAT
CTT TAT CAA ATT TAC GCA GAA TCT TTT AGA GAG TGG GAA GCA GAT  512
CCT ACT AAT CCA GCA TTA AGA GAA CAG ATG CGT ATT CAA TTC AAT
```

EP 0 186 379 B1

```
GAC ATG AAC AGT GCC CTT ACA ACC GCT ATT CCT CTT TTT GCA GTT   602

CAA AAT TAT CAA GTT CCT CTT TTA TCA GTA TAT GTT CAA GCT GCA

AAT TTA CAT TTA TCA GTT TTG AGA GAT GTT TCA GTG TTT GGA CAA   692

AGG TGG GGA TTT GAT GCC GCG ACT ATC AAT AGT CGT TAT AAT GAT

TTA ACT AGG CTT ATT GGC AAC TAT ACA GAT TAT GCT GTG CGC TGG   782

TAC AAT ACG GGA TTA GAG CGT GTA TGG GGA CCG GAT TCT AGA GAT

TGG GTA AGG TAT AAT CAA TTT AGA AGA GAG CTA ACA CTT ACT GTA   872

TTA GAT ATC CTT GCT CTA TTC TCA AAT TAT GAT AGT CGA AGG TAT

CCA ATT CGA ACA GTT TCC CAA TTA ACA AGA GAA ATT TAT ACG AAC   962

CCA GTA TTA GAA AAT TTT GAT GGT AGT TTT CGT GGA ATG GCT CAG

AGA ATA GAA CAG AAT ATT AGG CAA CCA CAT CTT ATG GAT ATC CTT   1052

AAT AGG ATA ACC ATT TAT ACT GAT GTG CAT AGA GGC TTT AAT TAT

TGG TCA GGG CAT CAA ATA ACA GCT TCT CCT GTA GGG TTT TCA GGA   1142

CCA GAA TTC GCA TTC CCT TTA TTT GGG AAT GCG GGG AAT GCA GCT

CCA CCC GTA CTT GTC TCA TTA ACT GGT TTG GGG ATT TTT AGA ACA   1232

TTA TCT TCA CCT TTA TAT AGA AGA ATT ATA CTT GGT TCA GGC CCA

AAT AAT CAG GAA CTG TTT GTC CTT GAT GGA ACG GAG TTT TCT TTT   1322

GCC TCC CTA ACG ACC AAC TTG CCT TCC ACT ATA TAT AGA CAA AGG

GGT ACA GTC GAT TCA CTA GAT GTA ATA CCG CCA CAG GAT AAT AGT   1412

GTA CCA CCT CGT GCG GGA TTT AGC CAT CGA TTG AGT CAT GTT ACA

ATG CTG AGC CAA GCA GCT GGA GCA GTT TAC ACC TTG AGA GCT CCA   1502

ACG TTT TCT TGG CAG CAT CGC AGT GCT GAA TTT AAT AAT ATA ATT

CCT TCA TCA CAA ATT ACA CAA ATA CCT TTA ACA AAA TCT ACT AAT   1592

CTT GGC TCT GGA ACT TCT GTC GTT AAA GGA CCA GGA TTT ACA GGA

GGA GAT ATT CTT CGA AGA ACT TCA CCT GGC CAG ATT TCA ACC TTA   1682

AGA GTA AAT ATT ACT GCA CCA TTA TCA CAA AGA TAT CGG GTA AGA

ATT CGC TAC GCT TCT ACT ACA AAT TTA CAA TTC CAT ACA TCA ATT   1772

GAC GGA AGA CCT ATT AAT CAG GGT AAT TTT TCA GCA ACT ATG AGT
```

```
AGT GGG AGT AAT TTA CAG TCC GGA AGC TTT AGG ACT GTA GGT TTT   1862

ACT ACT CCG TTT AAC TTT TCA AAT GGA TCA AGT GTA TTT ACG TTA

AGT GCT CAT GTC TTC AAT TCA GGC AAT GAA GTT TAT ATA GAT CGA   1952

ATT GAA TTT GTT CCG GCA GAA GTA ACC TTT GAG GCA GAA TAT GAT

TTA GAA AGA GCA CAA AAG GCG GTG AAT GAG CTG TTT ACT TCT TCC   2042

AAT CAA ATC GGG TTA AAA ACA GAT GTG ACG GAT TAT CAT ATT GAT

CAA GTA TCC AAT TTA GTT GAG TGT TTA TCA GAT GAA TTT TGT CTG   2132

GAT GAA AAA CAA GAA TTG TCC GAG AAA GTC AAA CAT GCG AAG CGA

CTT AGT GAT GAG CGG AAT TTA CTT CAA GAT CCA AAC TTC AGA GGG   2222

ATC AAT AGA CAA CTA GAC CGT GGC TGG AGA GGA AGT ACG GAT ATT

ACC ATC CAA GGA GGC GAT GAC GTA TTC AAA GAG AAT TAC GTT ACG   2312

CTA TTG GGT ACC TTT GAT GAG TGC TAT CCA ACG TAT TTA TAT CAA

AAA ATA GAT GAG TCG AAA TTA AAA GCC TAT ACC CGT TAT CAA TTA   2402

AGA GGG TAT ATC GAA GAT AGT CAA GAC TTA GAA ATC TAT TTA ATT

CGC TAC AAT GCA AAA CAT GAA ACA GTA AAT GTG CCA GGT ACG GGT   2492

TCC TTA TGG CCG CTT TCA GCC CAA AGT CCA ATC GGA AAG TGT GGA

GAG CCG AAT CGA TGC GCG CCA CAC CTT GAA TGG AAT CCT GAC TTA   2582

GAT TGT TCG TGT AGG GAT GGA GAA AAA TGT GCC CAT CAT TCC CAT

CAT TTC TCC TTG GAC ATT GAT GTT GGA TGT ACA GAC TTA AAT GAG   2672

GAC TTA GGT GTA TGG GTG ATA TTC AAG ATT AAG ACG CAA GAT GGC

CAT GCA AGA CTA GGA AAT CTA GAA TTT CTC GAA GAG AAA CCA TTA   2762

GTA GGA GAA GCA CTA GCT CGT GTG AAA AGA GCC GAG AAA AAA TGG

AGA GAC AAA CGT GAA AAA TTG GAA TGG GAA ACA AAT ATT GTT TAT   2852

AAA GAG GCA AAA GAA TCT GTA GAT GCT TTA TTT GTA AAC TCT CAA

TAT GAT AGA TTA CAA GCG GAT ACC AAC ATC GCG ATG ATT CAT GCG   2942

GCA GAT AAA CGC GTT CAT AGC ATT CGA GAA GCT TAT CTG CCT GAG

CTG TCT GTG ATT CCG GGT GTC AAT GCG GCT ATT TTT GAA GAA TTA   3032

GAA GGG CGT ATT TTC ACT GCA TTC TCC CTA TAT GAT GCG AGA AAT
```

GTC ATT AAA AAT GGT GAT TTT AAT AAT GGC TTA TCC TGC TGG AAC  3122

GTG AAA GGG CAT GTA GAT GTA GAA GAA CAA AAC AAC CAC CGT TCG

GTC CTT GTT GTT CCG GAA TGG GAA GCA GAA GTG TCA CAA GAA GTT  3212

CGT GTC TGT CCG GGT CGT GGC TAT ATC CTT CGT GTC ACA GCG TAC

AAG GAG GGA TAT GGA GAA GGT TGC GTA ACC ATT CAT GAG ATC GAG  3302

AAC AAT ACA GAC GAA CTG AAG TTT AGC AAC TGT GTA GAA GAG GAA

GTA TAT CCA AAC AAC ACG GTA ACG TGT AAT GAT TAT ACT GCG ACT  3392

CAA GAA GAA TAT GAG GGT ACG TAC ACT TCT CGT AAT CGA GGA TAT

GAC GGA GCC TAT GAA AGC AAT TCT TCT GTA CCA GCT GAT TAT GCA  3482

TCA GCC TAT GAA GAA AAA GCA TAT ACA GAT GGA CGA AGA GAC AAT

CCT TGT GAA TCT AAC AGA GGA TAT GGG GAT TAC ACA CCA CTA CCA  3572

GCT GGC TAT GTG ACA AAA GAA TTA GAG TAC TTC CCA GAA ACC GAT

AAG GTA TGG ATT GAG ATC GGA GAA ACG GAA GGA ACA TTC ATC GTG  3662

GAC AGC GTG GAA TTA CTT CTT ATG GAG GAA;

oder eine Sequenz, die mindestens 723 Codons von dem 5'-Endpunkt dieser Nucleotidsequenz aus enthält.

3. DNA-Fragment nach Anspruch 1 oder Anspruch 2, mit einer Länge von etwa 6,6 kb.

4. DNA-Fragment nach Anspruch 1, wobei die durch die Nucleotidsequenz codierte Aminosäuresequenz eine Aminosäuresequenz, beginnend mit Met an der Aminosäureposition 1 und beendet bei Arg an der Aminosäureposition 934, wie in Anspruch 1 definiert, enthält.

5. DNA-Fragment nach Anspruch 4, wobei die für die Aminosäuresequenz codierende Nucleotidsequenz eine Nucleotidsequenz, beginnend mit Position 153 und beendet an Position 2955, wie in Anspruch 2 definiert, enthält.

6. DNA-Fragment nach einem der Ansprüche 1, 4 und 6, mit einer Länge von etwa 2,95 kb.

7. Plasmid, das zum Reduplizieren in E. coli und zur Expression des in Anspruch 1 definierten Peptids befähigt ist, enthaltend ein Plasmid pBR322, bei den die in Anspruch 1 definierte DNA-Sequenz eingefügt ist.

8. Plasmid nach Anspruch 7, wobei das eingefügte DNA-Fragment eine Länge von etwa 6,6 kb besitzt.

9. Plasmid nach Anspruch 8, wobei das DNA-Fragment an der Pst I-Aufbrechungsstelle in das Plasmid pBR322 eingefügt ist.

10. Plasmid nach Anspruch 7, wobei das eingefügte DNA-Fragment eine Länge von etwa 2,95 kb besitzt.

11. Plasmid nach Anspruch 10, wobei das DNA-Fragment an der Bam HI-Aufbrechungsstelle in das Plasmid pBR322 eingefügt ist.

12. Mit einem Plasmid nach einem der Ansprüche 7 bis 11 transformierte und ein insektizides Protein erzeugende E. coli.

13. E. coli nach Anspruch 12, wobei das Protein ein Molekulargewicht von 144 kD hat.

14. E. coli nach Anspruch 12, wobei das Protein ein Molekulargewicht von 75 kD hat.

15. Insektizides Protein, das durch einen derart transformierten Organismus erzeugt ist, daß ein DNA-Fragment nach einem der Ansprüche 1 bis 6 eingebracht ist.

16. Zusammensetzung, enthaltend ein Protein nach Anspruch 15 in insektizid wirksamer Menge, zusammen mit einem Trägerstoff.

17. Verfahren zur Herstellung eines insektiziden Proteins, umfassend die Herstellung des Proteins durch einen Organismus, der derart transformiert wird, daß ein DNA-Fragment nach einem der Ansprüche 1 bis 6 eingebracht wird.

PATENTANSPRÜCHE (für der Vertgesstaat AT)

1. Verfahren, enthaltend die Herstellung eines DNA-Fragments, enthaltend eine für ein insektizides Peptid codierende Nucleotidsequenz mit der folgenden Aminosäuresequenz:

```
            1                                        10
Met Asp Asn Asn Pro Asn Ile Asn Glu Cys Ile Pro Tyr Asn Cys

                        20                               30
Leu Ser Asn Pro Glu Val Glu Val Leu Gly Gly Glu Arg Ile Glu

                                            40
Thr Gly Tyr Thr Pro Ile Asp Ile Ser Leu Ser Leu Thr Gln Phe

                        50                               60
Leu Leu Ser Glu Phe Val Pro Gly Ala Gly Phe Val Leu Gly Leu

                                            70
Val Asp Ile Ile Trp Gly Ile Phe Gly Pro Ser Gln Trp Asp Ala

                        80                               90
Phe Leu Val Gln Ile Glu Gln Leu Ile Asn Gln Arg Ile Glu Glu

                                            100
Phe Ala Arg Asn Gln Ala Ile Ser Arg Leu Glu Gly Leu Ser Asn

                        110                              120
Leu Tyr Gln Ile Tyr Ala Glu Ser Phe Arg Glu Trp Glu Ala Asp

                                            130
Pro Thr Asn Pro Ala Leu Arg Glu Glu Met Arg Ile Gln Phe Asn

                        140                              150
Asp Met Asn Ser Ala Leu Thr Thr Ala Ile Pro Leu Phe Ala Val

                                            160
Gln Asn Tyr Gln Val Pro Leu Leu Ser Val Tyr Val Gln Ala Ala

                        170                              180
Asn Leu His Leu Ser Val Leu Arg Asp Val Ser Val Phe Gly Gln

                                            190
Arg Trp Gly Phe Asp Ala Ala Thr Ile Asn Ser Arg Tyr Asn Asp

                        200                              210
Leu Thr Arg Leu Ile Gly Asn Tyr Thr Asp Tyr Ala Val Arg Trp
```

220
Tyr Asn Thr Gly Leu Glu Arg Val Trp Gly Pro Asp Ser Arg Asp

230                                              240
Trp Val Arg Try Asn Gln Phe Arg Arg Glu Leu Thr Leu Thr Val

250
Leu Asp Ile Val Ala Leu Phe Ser Asn Tyr Asp Ser Arg Arg Tyr

260                                              270
Pro Ile Arg Thr Val Ser Gln Leu Thr Arg Glu Ile Tyr Thr Asn

280
Pro Val leu Glu Asn Phe Asp Gly Ser Phe Arg Gly Met Ala Gln

290                                              300
Arg Ile Glu Gln Asn Ile Arg Gln Pro His Leu Met Asp Ile Leu

310
Asn Arg Ile Thr Ile Tyr Thr Asp Val His Arg Gly Phe Asn Tyr

320                                              330
Trp Ser Gly His Gln Ile Thr Ala Ser Pro Val Gly Phe Ser Gly

340
Pro Glu Phe Ala Phe Pro Leu Phe Gly Asn Ala Gly Asn Ala Ala

350                                              360
Pro Pro Val Leu Val Ser Leu Thr Gly Leu Gly Ile Phe Arg Thr

370
Leu Ser Ser Pro Leu Tyr Arg Arg Ile Ile Leu Gly Ser Gly Pro

380                                              390
Asn Asn Gln Glu Leu Phe Val Leu Asp Gly Thr Glu Phe Ser Phe

400
Ala Ser Leu Thr Thr Asn Leu Pro Ser Thr Ile Tyr Arg Gln Arg

·410                                             420
Gly Thr Val Asp Ser Leu Asp Val Ile Pro Pro Gln Asp Asn Ser

430
Val Pro Pro Arg Ala Gly Phe Ser His Arg Leu Ser His Val Thr

440                                              450
Met Leu Ser Gln Ala Ala Gly Ala Val Tyr Thr Leu Arg Ala Pro

460
Thr Phe Ser Trp Gln His Arg Ser Ala Glu Phe Asn Asn Ile Ile

470                                              480
Pro Ser Ser Gln Ile Thr Gln Ile Pro Leu Thr Lys Ser Thr Asn

490
Leu Gly Ser Gly Thr Ser Val Val Lys Gly Pro Gly Phe Thr Gly

500                                                   510
Gly Asp Ile Leu Arg Arg Thr Ser Pro Gly Gln Ile Ser Thr Leu

520
Arg Val Asn Ile Thr Ala Pro Leu Ser Gln Arg Tyr Arg Val Arg

530                                                   540
Ile Arg Tyr Ala Ser Thr Thr Asn Leu Gln Phe His Thr Ser Ile

550
Asp Gly Arg Pro Ile Asn Gln Gly Asn Phe Ser Ala Thr Met Ser

560                                                   570
Ser Gly Ser Asn Leu Gln Ser Gly Ser Phe Arg Thr Val Gly Phe

580
Thr Thr Pro Phe Asn Phe Ser Asn Gly Ser Ser Val Phe Thr Leu

590                                                   600
Ser Ala His Val Phe Asn Ser Gly Asn Glu Val Tyr Ile Asp Arg

610
Ile Glu Phe Val Pro Ala Glu Val Thr Phe Glu Ala Glu Tyr Asp

620                                                   630
Leu Glu Arg Ala Gln Lys Ala Val Asn Glu Leu Phe Thr Ser Ser

640
Asn Gln Ile Gly Leu Lys Thr Asp Val Thr Asp Tyr His Ile Asp

650                                                   660
Gln Val Ser Asn Leu Val Glu Cys Leu Ser Asp Glu Phe Cys Leu

670
Asp Glu Lys Gln Glu Leu Ser Glu Lys Val Lys His Ala Lys Arg

680                                                   690
Leu Ser Asp Glu Arg Asn Leu Leu Gln Asp Pro Asn Phe Arg Gly

700
Ile Asn Arg Gln Leu Asp Arg Gly Trp Arg Gly Ser Thr Asp Ile

710                                                   720
Thr Ile Gln Gly Gly Asp Asp Val Phe Lys Glu Asn Tyr Val Thr

730
Leu Leu Gly Thr Phe Asp Glu Cys Tyr Pro Thr Tyr Leu Tyr Gln

740                                                   750
Lys Ile Asp Glu Ser Lys Leu Lys Ala Tyr Thr Arg Tyr Gln Leu

760
Arg Gly Tyr Ile Glu Asp Ser Gln Asp Leu Glu Ile Tyr Leu Ile

770                                             780
Arg Tyr Asn Ala Lys His Glu Thr Val Asn Val Pro Gly Thr Gly

790
Ser Leu Trp Pro Leu Ser Ala Gln Ser Pro Ile Gly Lys Cys Gly

800                                             810
Glu Pro Asn Arg Cys Ala Pro His Leu Glu Trp Asn Pro Asp Leu

820
Asp Cys Ser Cys Arg Asp Gly Glu Lys Cys Ala His His Ser His

830                                             840
His Phe Ser Leu Asp Ile Asp Val Gly Cys Thr Asp Leu Asn Glu

850
Asp Leu Gly Val Trp Val Ile Phe Lys Ile Lys Thr Gln Asp Gly

860                                             870
His Ala Arg Leu Gly Asn Leu Glu Phe Leu Glu Glu Lys Pro Leu

880
Val Gly Glu Ala Leu Ala Arg Val Lys Arg Ala Glu Lys Lys Trp

890                                             900
Arg Asp Lys Arg Glu Lys Leu Glu Trp Glu Thr Asn Ile Val Tyr

910
Lys Glu Ala Lys Glu Ser Val Asp Ala Leu Phe Val Asn Ser Gln

920                                             930
Tyr Asp Arg Leu Gln Ala Asp Thr Asn Ile Ala Met Ile His Ala

940
Ala Asp Lys Arg Val His Ser Ile Arg Glu Ala Tyr Leu Pro Glu

950                                             960
Leu Ser Val Ile Pro Gly Val Asn Ala Ala Ile Phe Glu Glu Leu

970
Glu Gly Arg Ile Phe Thr Ala Phe Ser Leu Tyr Asp Ala Arg Asn

980                                             990
Val Ile Lys Asn Gly Asp Phe Asn Asn Gly Leu Ser Cys Trp Asn

1000
Val Lys Gly His Val Asp Val Glu Glu Gln Asn Asn His Arg Ser

1010                                            1020
Val Leu Val Val Pro Glu Trp Glu Ala Glu Val Ser Gln Glu Val

1030
Arg Val Cys Pro Gly Arg Gly Tyr Ile Leu Arg Val Thr Ala Tyr

1040                                                                1050
Lys Glu Gly Tyr Gly Glu Gly Cys Val Thr Ile His Glu Ile Glu

1060
Asn Asn Thr Asp Glu Leu Lys Phe Ser Asn Cys Val Glu Glu Glu

1070                                                                1080
Val Tyr Pro Asn Asn Thr Val Thr Cys Asn Asp Tyr Thr Ala Thr

1090
Gln Glu Glu Tyr Glu Gly Thr Tyr Thr Ser Arg Asn Arg Gly Tyr

1100                                                                1110
Asp Gly Ala Tyr Glu Ser Asn Ser Ser Val Pro Ala Asp Tyr Ala

1120
Ser Ala Tyr Glu Glu Lys Ala Tyr Thr Asp Gly Arg Arg Asp Asn

1130                                                                1140
Pro Cys Glu Ser Asn Arg Gly Tyr Gly Asp Tyr Thr Pro Leu Pro

1150
Ala Gly Tyr Val Thr Lys Glu Leu Glu Tyr Phe Pro Glu Thr Asp

1160                                                                1170
Lys Val Trp Ile Glu Ile Gly Glu Thr Glu Gly Thr Phe Ile Val

1180
Asp Ser Val Glu Leu Leu Leu Met Glu Glu,

oder eine Aminosäuresequenz, die mindestens 723 Aminosäuren von dem N-Endpunkt dieser Aminosäuresequenz aus enthält.

2. Verfahren nach Anspruch 1, wobei die Nucleotidsequenz die folgende Sequenz enthält:

```
ATG GAT AAC AAT CCG AAC ATC AAT GAA TGC ATT CCT TAT AAT TGT

TTA AGT AAC CCT GAA GTA GAA GTA TTA GGT GGA GAA AGA GTG GAA      242

ACT GGT TAC ACC CCA ATC GAT ATT TCC TTG TCG CTA ACG CAA TTT

CTT TTG AGT GAA TTT GTT CCC GGT GCT GGA TTT GTG TTA GGA CTA      332

GTT GAT ATA ATA TGG GGA ATT TTT GGT CCC TCT CAA TGG GAC GCA

TTT CTT GTA CAA ATT GAA CAG TTA ATT AAC CAA AGA ATA GAA GAA      422

TTC GCT AGG AAC CAA GCC ATT TCT AGA TTA GAA GGA CTA AGC AAT

CTT TAT CAA ATT TAC GCA GAA TCT TTT AGA GAG TGG GAA GCA GAT      512

CCT ACT AAT CCA GCA TTA AGA GAA GAG ATG CGT ATT CAA TTC AAT
```

```
GAC ATG AAC AGT GCC CTT ACA ACC GCT ATT CCT CTT TTT GCA GTT   602

CAA AAT TAT CAA GTT CCT CTT TTA TCA GTA TAT GTT CAA GCT GCA

AAT TTA CAT TTA TCA GTT TTG AGA GAT GTT TCA GTG TTT GGA CAA   692

AGG TGG GGA TTT GAT GCC GCG ACT ATC AAT AGT CGT TAT AAT GAT

TTA ACT AGG CTT ATT GGC AAC TAT ACA GAT TAT GCT GTG CGC TGG   782

TAC AAT ACG GGA TTA GAG CGT GTA TGG GGA CCG GAT TCT AGA GAT

TGG GTA AGG TAT AAT CAA TTT AGA AGA GAG CTA ACA CTT ACT GTA   872

TTA GAT ATC CTT GCT CTA TTC TCA AAT TAT GAT AGT CGA AGG TAT

CCA ATT CGA ACA GTT TCC CAA TTA ACA AGA GAA ATT TAT ACG AAC   962

CCA GTA TTA GAA AAT TTT GAT GGT AGT TTT CGT GGA ATG GCT CAG

AGA ATA GAA CAG AAT ATT AGG CAA CCA CAT CTT ATG GAT ATC CTT   1052

AAT AGG ATA ACC ATT TAT ACT GAT GTG CAT AGA GGC TTT AAT TAT

TGG TCA GGG CAT CAA ATA ACA GCT TCT CCT GTA GGG TTT TCA GGA   1142

CCA GAA TTC GCA TTC CCT TTA TTT GGG AAT GCG GGG AAT GCA GCT

CCA CCC GTA CTT GTC TCA TTA ACT GGT TTG GGG ATT TTT AGA ACA   1232

TTA TCT TCA CCT TTA TAT AGA AGA ATT ATA CTT GGT TCA GGC CCA

AAT AAT CAG GAA CTG TTT GTC CTT GAT GGA ACG GAG TTT TCT TTT   1322

GCC TCC CTA ACG ACC AAC TTG CCT TCC ACT ATA TAT AGA CAA AGG

GGT ACA GTC GAT TCA CTA GAT GTA ATA CCG CCA CAG GAT AAT AGT   1412

GTA CCA CCT CGT GCG GGA TTT AGC CAT CGA TTG AGT CAT GTT ACA

ATG CTG AGC CAA GCA GCT GGA GCA GTT TAC ACC TTG AGA GCT CCA   1502

ACG TTT TCT TGG CAG CAT CGC AGT GCT GAA TTT AAT AAT ATA ATT

CCT TCA TCA CAA ATT ACA CAA ATA CCT TTA ACA AAA TCT ACT AAT   1592

CTT GGC TCT GGA ACT TCT GTC GTT AAA GGA CCA GGA TTT ACA GGA

GGA GAT ATT CTT CGA AGA ACT TCA CCT GGC GAG ATT TCA ACC TTA   1682

AGA GTA AAT ATT ACT GCA CCA TTA TCA CAA AGA TAT CGG GTA AGA

ATT CGC TAC GCT TCT ACT ACA AAT TTA CAA TTC CAT ACA TCA ATT   1772

GAC GGA AGA CCT ATT AAT CAG GGT AAT TTT TCA GCA ACT ATG AGT
```

AGT GGG AGT AAT TTA CAG TCC GGA AGC TTT AGG ACT GTA GGT TTT    1862

ACT ACT CCG TTT AAC TTT TCA AAT GGA TCA AGT GTA TTT ACG TTA

AGT GCT CAT GTC TTC AAT TCA GGC AAT GAA GTT TAT ATA GAT CGA    1952

ATT GAA TTT GTT CCG GCA GAA GTA ACC TTT GAG GCA GAA TAT GAT

TTA GAA AGA GCA CAA AAG GCG GTG AAT GAG CTG TTT ACT TCT TCC    2042

AAT CAA ATC GGG TTA AAA ACA GAT GTG ACG GAT TAT CAT ATT GAT

CAA GTA TCC AAT TTA GTT GAG TGT TTA TCA GAT GAA TTT TGT CTG    2132

GAT GAA AAA CAA GAA TTG TCC GAG AAA GTC AAA CAT GCG AAG CGA

CTT AGT GAT GAG CGG AAT TTA CTT CAA GAT CCA AAC TTC AGA GGG    2222

ATC AAT AGA CAA CTA GAC CGT GGC TGG AGA GGA AGT ACG GAT ATT

ACC ATC CAA GGA GGC GAT GAC GTA TTC AAA GAG AAT TAC GTT ACG    2312

CTA TTG GGT ACC TTT GAT GAG TGC TAT CCA ACG TAT TTA TAT CAA

AAA ATA GAT GAG TCG AAA TTA AAA GCC TAT ACC CGT TAT CAA TTA    2402

AGA GGG TAT ATC GAA GAT AGT CAA GAC TTA GAA ATC TAT TTA ATT

CGC TAC AAT GCA AAA CAT GAA ACA GTA AAT GTG CCA GGT ACG GGT    2492

TCC TTA TGG CCG CTT TCA GCC CAA AGT CCA ATC GGA AAG TGT GGA

GAG CCG AAT CGA TGC GCG CCA CAC CTT GAA TGG AAT CCT GAC TTA    2582

GAT TGT TCG TGT AGG GAT GGA GAA AAA TGT GCC CAT CAT TCC CAT

CAT TTC TCC TTG GAC ATT GAT GTT GGA TGT ACA GAC TTA AAT GAG    2672

GAC TTA GGT GTA TGG GTG ATA TTC AAG ATT AAG ACG CAA GAT GGC

CAT GCA AGA CTA GGA AAT CTA GAA TTT CTC GAA GAG AAA CCA TTA    2762

GTA GGA GAA GCA CTA GCT CGT GTG AAA AGA GCC GAG AAA AAA TGG

AGA GAC AAA CGT GAA AAA TTG GAA TGG GAA ACA AAT ATT GTT TAT    2852

AAA GAG GCA AAA GAA TCT GTA GAT GCT TTA TTT GTA AAC TCT CAA

TAT GAT AGA TTA CAA GCG GAT ACC AAC ATC GCG ATG ATT CAT GCG    2942

GCA GAT AAA CGC GTT CAT AGC ATT CGA GAA GCT TAT CTG CCT GAG

CTG TCT GTG ATT CCG GGT GTC AAT GCG GCT ATT TTT GAA GAA TTA    3032

GAA GGG CGT ATT TTC ACT GCA TTC TCC CTA TAT GAT GCG AGA AAT

```
GTC ATT AAA AAT GGT GAT TTT AAT AAT GGC TTA TCC TGC TGG AAC   3122

GTG AAA GGG CAT GTA GAT GTA GAA GAA CAA AAC AAC CAC CGT TCG

GTC CTT GTT GTT CCG GAA TGG GAA GCA GAA GTG TCA CAA GAA GTT   3212

CGT GTC TGT CCG GGT CGT GGC TAT ATC CTT CGT GTC ACA GCG TAC

AAG GAG GGA TAT GGA GAA GGT TGC GTA ACC ATT CAT GAG ATC GAG   3302

AAC AAT ACA GAC GAA CTG AAG TTT AGC AAC TGT GTA GAA GAG GAA

GTA TAT CCA AAC AAC ACG GTA ACG TGT AAT GAT TAT ACT GCG ACT   3392

CAA GAA GAA TAT GAG GGT ACG TAC ACT TCT CGT AAT CGA GGA TAT

GAC GGA GCC TAT GAA AGC AAT TCT TCT GTA CCA GCT GAT TAT GCA   3482

TCA GCC TAT GAA GAA AAA GCA TAT ACA GAT GGA CGA AGA GAC AAT

CCT TGT GAA TCT AAC AGA GGA TAT GGG GAT TAC ACA CCA CTA CCA   3572

GCT GGC TAT GTG ACA AAA GAA TTA GAG TAC TTC CCA GAA ACC GAT

AAG GTA TGG ATT GAG ATC GGA GAA ACG GAA GGA ACA TTC ATC GTG   3662

GAC AGC GTG GAA TTA CTT CTT ATG GAG GAA,
```

oder eine Sequenz, die mindestens 723 Codons von dem 5'-Endpunkt dieser Nucleotidsequenz aus enthält.

3. Verfahren nach Anspruch 1 oder 2, wobei das DNA-Fragment eine Länge von etwa 6,6 kb besitzt.

4. Verfahren nach Anspruch 1, wobei die durch die Nucleotidsequenz codierte Aminosäuresequenz eine Aminosäuresequenz, beginnend mit Met an der Aminosäureposition 1 und beendet mit Arg an der Aminosäureposition 934, wie in Anspruch 1 definiert, enthält.

5. Verfahren nach Anspruch 4, wobei die für die Aminosäuresequenz codierende Nucleotidsequenz eine Nucleotidsequenz, beginnend bei Position 153 und beendet bei Position 2955, wie in Anspruch 2 definiert, enthält.

6. Verfahren nach den Ansprüchen 1, 4 und 5, wobei das DNA-Fragment eine Länge von etwa 2,95 kb besitzt.

7. Verfahren, umfassend die Herstellung eines Plasmids, das zum Reduplizieren in E. coli und zur Expression des in Anspruch 1 definierten Peptids befähigt ist, enthaltend das Einfügen einer DNA-Sequenz, wie in Anspruch 1 definiert, in ein Plasmid pBR322.

8. Verfahren nach Anspruch 7, wobei das eingefügte DNA-Fragment eine Länge von etwa 6,6 kb besitzt.

9. Verfahren nach Anspruch 8, wobei das DNA-Fragment an der Pst I-Aufbrechungsstelle in das Plasmid pBR322 eingefügt ist.

10. Verfahren nach Anspruch 7, wobei das eingefügte DNA-Fragment eine Länge von etwa 2,95 kg besitzt.

**11.** Verfahren nach Anspruch 10, wobei das DNA-Fragment an der Bam HI-Aufbrechungsstelle in das Plasmid pBR322 eingefügt ist.

**12.** Mit einem Plasmid, hergestellt nach einem der Ansprüche 7 bis 11, transformierte und ein insektizides Protein erzeugende E. coli.

**13.** E. coli nach Anspruch 12, wobei das Protein ein Molekulargewicht von 144 kD besitzt.

**14.** E. coli nach Anspruch 12, wobei das Protein ein Molekulargewicht von 75 kD besitzt.

**15.** Verfahren zur Herstellung eines insektiziden Proteins, enthaltend die Herstellung des Proteins durch einen derart transformierten Organismus, daß ein DNA-Fragment nach einem der Ansprüche 1 bis 6 eingebracht wird.

**16.** Verfahren nach Anspruch 15, wobei der Organismus E. coli nach einem der Ansprüche 12, 13 und 14 ist.

## Fig. I-1

AACACCCTGGGTCAAAAATT    20

GATATTTAGTAAAATTAGTTGCACTTTGTGCATTTTTTCATAAGATGAGTCATATGTTTTAAATTG    86

TAGTAATGAAAAACAGTATTATATCATAATGAATTGGTATCTTAATAAAAGAGATGGAGGTAACTT    152

```
                                10                                20
ATG GAT AAC AAT CCG AAC ATC AAT GAA TGC ATT CCT TAT AAT TGT TTA AGT AAC CCT GAA    212
Met Asp Asn Asn Pro Asn Ile Asn Glu Cys Ile Pro Tyr Asn Cys Leu Ser Asn Pro Glu

                                30                                40
GTA GAA GTA TTA GGT GGA GAA AGA ATA GAA ACT GGT TAC ACC CCA ATC GAT ATT TCC TTG    272
Val Glu Val Leu Gly Gly Glu Arg Ile Glu Thr Gly Tyr Thr Pro Ile Asp Ile Ser Leu

                                50                                60
TCG CTA ACG CAA TTT CTT TTG AGT GAA TTT GTT CCC GGT GCT GGA TTT GTG TTA GGA CTA    332
Ser Leu Thr Gln Phe Leu Leu Ser Glu Phe Val Pro Gly Ala Gly Phe Val Leu Gly Leu

                                70                                80
GTT GAT ATA ATA TGG GGA ATT TTT GGT CCC TCT CAA TGG GAC GCA TTT CTT GTA CAA ATT    392
Val Asp Ile Ile Trp Gly Ile Phe Gly Pro Ser Gln Trp Asp Ala Phe Leu Val Gln Ile

                                90                                100
GAA CAG TTA ATT AAC CAA AGA ATA GAA GAA TTC GCT AGG AAC CAA GCC ATT TCT AGA TTA    452
Glu Gln Leu Ile Asn Gln Arg Ile Glu Glu Phe Ala Arg Asn Gln Ala Ile Ser Arg Leu
```

EP 0 186 379 B1

## Fig. I-2

```
                                           110                                              120
GAA GGA CTA AGC AAT CTT TAT CAA ATT TAC GCA GAA TCT TTT AGA GAG TGG GAA GCA GAT    512
Glu Gly Leu Ser Asn Leu Tyr Gln Ile Tyr Ala Glu Ser Phe Arg Glu Trp Glu Ala Asp

121                                        130                                              140
CCT ACT AAT CCA GCA TTA AGA GAA GAG ATG CGT ATT CAA TTC AAT GAC ATG AAC AGT GCC    572
Pro Thr Asn Pro Ala Leu Arg Glu Glu Met Arg Ile Gln Phe Asn Asp Met Asn Ser Ala

                                           150                                              160
CTT ACA ACC GCT ATT CCT CTT TTT GCA GTT CAA AAT TAT CAA GTT CCT CTT TTA TCA GTA    632
Leu Thr Thr Ala Ile Pro Leu Phe Ala Val Gln Asn Tyr Gln Val Pro Leu Leu Ser Val

                                           170                                              180
TAT GTT CAA GCT GCA AAT TTA CAT TTA TCA GTT TTG AGA GAT GTT TCA GTG TTT GGA CAA    692
Tyr Val Gln Ala Ala Asn Leu His Leu Ser Val Leu Arg Asp Val Ser Val Phe Gly Gln

                                           190                                              200
AGG TGG GGA TTT GAT GCC GCG ACT ATC AAT AGT CGT TAT AAT GAT TTA ACT AGG CTT ATT    752
Arg Trp Gly Phe Asp Ala Ala Thr Ile Asn Ser Arg Tyr Asn Asp Leu Thr Arg Leu Ile

                                           210                                              220
GGC AAC TAT ACA GAT TAT GCT GTG CGC TGG TAC AAT ACG GGA TTA GAG CGT GTA TGG GGA    812
Gly Asn Tyr Thr Asp Tyr Ala Val Arg Trp Tyr Asn Thr Gly Leu Glu Arg Val Trp Gly

                                           230                                              240
CCG GAT TCT AGA GAT TGG GTA AGG TAT AAT CAA TTT AGA AGA GAG CTA ACA CTT ACT GTA    872
Pro Asp Ser Arg Asp Trp Val Arg Tyr Asn Gln Phe Arg Arg Glu Leu Thr Leu Thr Val

                                           250                                              260
TTA GAT ATC GTT GCT CTA TTC TCA AAT TAT GAT AGT CGA AGG TAT CCA ATT CGA ACA GTT    932
Leu Asp Ile Val Ala Leu Phe Ser Asn Tyr Asp Ser Arg Arg Tyr Pro Ile Arg Thr Val
```

EP 0 186 379 B1

# Fig. I-3

EP 0 186 379 B1

```
                                    270                                    280
TCC CAA TTA ACA AGA GAA ATT TAT ACG AAC CCA GTA TTA GAA AAT TTT GAT GGT AGT TTT   992
Ser Gln Leu Thr Arg Glu Ile Tyr Thr Asn Pro Val Leu Glu Asn Phe Asp Gly Ser Phe

                                    290                                    300
CGT GGA ATG GCT CAG AGA ATA GAA CAG AAT ATT AGG CAA CCA CAT CTT ATG GAT ATC CTT  1052
Arg Gly Met Ala Gln Arg Ile Glu Gln Asn Ile Arg Gln Pro His Leu Met Asp Ile Leu

                                    310                                    320
AAT AGG ATA ACC ATT TAT ACT GAT GTG CAT AGA GGC TTT AAT TAT TGG TCA GGG CAT CAA  1112
Asn Arg Ile Thr Ile Tyr Thr Asp Val His Arg Gly Phe Asn Tyr Trp Ser Gly His Gln

                                    330                                    340
ATA ACA GCT TCT CCT GTA GGG TTT TCA GGA CCA GAA TTC GCA TTC CCT TTA TTT GGG AAT  1172
Ile Thr Ala Ser Pro Val Gly Phe Ser Gly Pro Glu Phe Ala Phe Pro Leu Phe Gly Asn

                                    350                                    360
GCG GGG AAT GCA GCT CCA CCC GTA CTT GTC TCA TTA ACT GGT TTG GGG ATT TTT AGA ACA  1232
Ala Gly Asn Ala Ala Pro Pro Val Leu Val Ser Leu Thr Gly Leu Gly Ile Phe Arg Thr

361                                 370                                    380
TTA TCT TCA CCT TTA TAT AGA AGA ATT ATA CTT GGT TCA GGC CCA AAT AAT CAG GAA CTG  1292
Leu Ser Ser Pro Leu Tyr Arg Arg Ile Ile Leu Gly Ser Gly Pro Asn Asn Gln Glu Leu

                                    390                                    400
TTT GTC CTT GAT GGA ACG GAG TTT TCT TTT GCC TCC CTA ACG ACC AAC TTG CCT TCC ACT  1352
Phe Val Leu Asp Gly Thr Glu Phe Ser Phe Ala Ser Leu Thr Thr Asn Leu Pro Ser Thr

                                    410                                    420
ATA TAT AGA CAA AGG GGT ACA GTC GAT TCA CTA GAT GTA ATA CCG CCA CAG GAT AAT AGT  1412
Ile Tyr Arg Gln Arg Gly Thr Val Asp Ser Leu Asp Val Ile Pro Pro Gln Asp Asn Ser
```

## Fig. 1-4

```
                              430                                   440
GTA CCA CCT CGT GCG GGA TTT AGC CAT CGA TTG AGT CAT GTT ACA ATG CTG AGC CAA GCA   1472
Val Pro Pro Arg Ala Gly Phe Ser His Arg Leu Ser His Val Thr Met Leu Ser Gln Ala

                              450                                   460
GCT GGA GCA GTT TAC ACC TTG AGA GCT CCA ACG TTT TCT TGG CAG CAT CGC AGT GCT GAA   1532
Ala Gly Ala Val Tyr Thr Leu Arg Ala Pro Thr Phe Ser Trp Gln His Arg Ser Ala Glu

                              470                                   480
TTT AAT AAT ATA ATT CCT TCA TCA CAA ATT ACA CAA ATA CCT TTA ACA AAA TCT ACT AAT   1592
Phe Asn Asn Ile Ile Pro Ser Ser Gln Ile Thr Gln Ile Pro Leu Thr Lys Ser Thr Asn

481                           490                                   500
CTT GGC TCT GGA ACT TCT GTC GTT AAA GGA CCA GGA TTT ACA GGA GGA GAT ATT CTT CGA   1652
Leu Gly Ser Gly Thr Ser Val Val Lys Gly Pro Gly Phe Thr Gly Gly Asp Ile Leu Arg

                              510                                   520
AGA ACT TCA CCT GGC CAG ATT TCA ACC TTA AGA GTA AAT ATT ACT GCA CCA TTA TCA CAA   1712
Arg Thr Ser Pro Gly Gln Ile Ser Thr Leu Arg Val Asn Ile Thr Ala Pro Leu Ser Gln

                              530                                   540
AGA TAT CGG GTA AGA ATT CGC TAC GCT TCT ACT ACA AAT TTA CAA TTC CAT ACA TCA ATT   1772
Arg Tyr Arg Val Arg Ile Arg Tyr Ala Ser Thr Thr Asn Leu Gln Phe His Thr Ser Ile

                              550                                   560
GAC GGA AGA CCT ATT AAT CAG GGT AAT TTT TCA GCA ACT ATG AGT AGT GGG AGT AAT TTA   1832
Asp Gly Arg Pro Ile Asn Gln Gly Asn Phe Ser Ala Thr Met Ser Ser Gly Ser Asn Leu

                              570                                   580
CAG TCC GGA AGC TTT AGG ACT GTA GGT TTT ACT ACT CCG TTT AAC TTT TCA AAT GGA TCA   1892
Gln Ser Gly Ser Phe Arg Thr Val Gly Phe Thr Thr Pro Phe Asn Phe Ser Asn Gly Ser
```

EP 0 186 379 B1

# Fig. 1-5

```
                                      590                                    600
AGT GTA TTT ACG TTA AGT GCT CAT GTC TTC AAT TCA GGC AAT GAA GTT TAT ATA GAT CGA   1952
Ser Val Phe Thr Leu Ser Ala His Val Phe Asn Ser Gly Asn Glu Val Tyr Ile Asp Arg

                                      610                                    620
ATT GAA TTT GTT CCG GCA GAA GTA ACC TTT GAG GCA GAA TAT GAT TTA GAA AGA GCA CAA   2012
Ile Glu Phe Val Pro Ala Glu Val Thr Phe Glu Ala Glu Tyr Asp Leu Glu Arg Ala Gln

                                      630                                    640
AAG GCG GTG AAT GAG CTG TTT ACT TCT TCC AAT CAA ATC GGG TTA AAA ACA GAT GTG ACG   2072
Lys Ala Val Asn Glu Leu Phe Thr Ser Ser Asn Gln Ile Gly Leu Lys Thr Asp Val Thr

                                      650                                    660
GAT TAT CAT ATT GAT CAA GTA TCC AAT TTA GTT GAG TGT TTA TCA GAT GAA TTT TGT CTG   2132
Asp Tyr His Ile Asp Gln Val Ser Asn Leu Val Glu Cys Leu Ser Asp Glu Phe Cys Leu

                                      670                                    680
GAT GAA AAA CAA GAA TTG TCC GAG AAA GTC AAA CAT GCG AAG CGA CTT AGT GAT GAG CGG   2192
Asp Glu Lys Gln Glu Leu Ser Glu Lys Val Lys His Ala Lys Arg Leu Ser Asp Glu Arg

                                      690                                    700
AAT TTA CTT CAA GAT CCA AAC TTC AGA GGG ATC AAT AGA CAA CTA GAC CGT GGC TGG AGA   2252
Asn Leu Leu Gln Asp Pro Asn Phe Arg Gly Ile Asn Arg Gln Leu Asp Arg Gly Trp Arg

                                      710                                    720
GGA AGT ACG GAT ATT ACC ATC CAA GGA GGC GAT GAC GTA TTC AAA GAG AAT TAC GTT ACG   2312
Gly Ser Thr Asp Ile Thr Ile Gln Gly Gly Asp Asp Val Phe Lys Glu Asn Tyr Val Thr

                                      730                                    740
CTA TTG GGT ACC TTT GAT GAG TGC TAT CCA ACG TAT TTA TAT CAA AAA ATA GAT GAG TCG   2372
Leu Leu Gly Thr Phe Asp Glu Cys Tyr Pro Thr Tyr Leu Tyr Gln Lys Ile Asp Glu Ser
```

## Fig. I-6

```
                                    750                                              760
AAA TTA AAA GCC TAT ACC CGT TAT CAA TTA AGA GGG TAT ATC GAA GAT AGT CAA GAC TTA   2432
Lys Leu Lys Ala Tyr Thr Arg Tyr Gln Leu Arg Gly Tyr Ile Glu Asp Ser Gln Asp Leu


                                    770                                              780
GAA ATC TAT TTA ATT CGC TAC AAT GCA AAA CAT GAA ACA GTA AAT GTG CCA GGT ACG GGT   2492
Glu Ile Tyr Leu Ile Arg Tyr Asn Ala Lys His Glu Thr Val Asn Val Pro Gly Thr Gly


                                    790                                              800
TCC TTA TGG CCG CTT TCA GCC CAA AGT CCA ATC GGA AAG TGT GGA GAG CCG AAT CGA TGC   2552
Ser Leu Trp Pro Leu Ser Ala Gln Ser Pro Ile Gly Lys Cys Gly Glu Pro Asn Arg Cys


                                    810                                              820
GCG CCA CAC CTT GAA TGG AAT CCT GAC TTA GAT TGT TCG TGT AGG GAT GGA GAA AAA TGT   2612
Ala Pro His Leu Glu Trp Asn Pro Asp Leu Asp Cys Ser Cys Arg Asp Gly Glu Lys Cys


                                    830                                              840
GCC CAT CAT TCC CAT CAT TTC TCC TTG GAC ATT GAT GTT GGA TGT ACA GAC TTA AAT GAG   2672
Ala His His Ser His His Phe Ser Leu Asp Ile Asp Val Gly Cys Thr Asp Leu Asn Glu


                                    850                                              860
GAC TTA GGT GTA TGG GTG ATA TTC AAG ATT AAG ACG CAA GAT GGC CAT GCA AGA CTA GGA   2732
Asp Leu Gly Val Trp Val Ile Phe Lys Ile Lys Thr Gln Asp Gly His Ala Arg Leu Gly


                                    870                                              880
AAT CTA GAA TTT CTC GAA GAG AAA CCA TTA GTA GGA GAA GCA CTA GCT CGT GTG AAA AGA   2792
Asn Leu Glu Phe Leu Glu Glu Lys Pro Leu Val Gly Glu Ala Leu Ala Arg Val Lys Arg


                                    890                                              900
GCC GAG AAA AAA TGG AGA GAC AAA CGT GAA AAA TTG GAA TGG GAA ACA AAT ATT GTT TAT   2852
Ala Glu Lys Lys Trp Arg Asp Lys Arg Glu Lys Leu Glu Trp Glu Thr Asn Ile Val Tyr
```

EP 0 186 379 B1

Fig. I-7

```
                                    910                                          920
AAA GAG GCA AAA GAA TCT GTA GAT GCT TTA TTT GTA AAC TCT CAA TAT GAT AGA TTA CAA   2912
Lys Glu Ala Lys Glu Ser Val Asp Ala Leu Phe Val Asn Ser Gln Tyr Asp Arg Leu Gln


                                    930                                          940
GCG GAT ACC AAC ATC GCG ATG ATT CAT GCG GCA GAT AAA CGC GTT CAT AGC ATT CGA GAA   2972
Ala Asp Thr Asn Ile Ala Met Ile His Ala Ala Asp Lys Arg Val His Ser Ile Arg Glu


                                    950                                          960
GCT TAT CTG CCT GAG CTG TCT GTG ATT CCG GGT GTC AAT GCG GCT ATT TTT GAA GAA TTA   3032
Ala Tyr Leu Pro Glu Leu Ser Val Ile Pro Gly Val Asn Ala Ala Ile Phe Glu Glu Leu


                                    970                                          980
GAA GGG CGT ATT TTC ACT GCA TTC TCC CTA TAT GAT GCG AGA AAT GTC ATT AAA AAT GGT   3092
Glu Gly Arg Ile Phe Thr Ala Phe Ser Leu Tyr Asp Ala Arg Asn Val Ile Lys Asn Gly


                                    990                                         1000
GAT TTT AAT AAT GGC TTA TCC TGC TGG AAC GTG AAA GGG CAT GTA GAT GTA GAA GAA CAA   3152
Asp Phe Asn Asn Gly Leu Ser Cys Trp Asn Val Lys Gly His Val Asp Val Glu Glu Gln


                                   1010                                         1020
AAC AAC CAC CGT TCG GTC CTT GTT GTT CCG GAA TGG GAA GCA GAA GTG TCA CAA GAA GTT   3212
Asn Asn His Arg Ser Val Leu Val Val Pro Glu Trp Glu Ala Glu Val Ser Gln Glu Val


                                   1030                                         1040
CGT GTC TGT CCG GGT CGT GGC TAT ATC CTT CGT GTC ACA GCG TAC AAG GAG GGA TAT GGA   3272
Arg Val Cys Pro Gly Arg Gly Tyr Ile Leu Arg Val Thr Ala Tyr Lys Glu Gly Tyr Gly


                                   1050                                         1060
GAA GGT TGC GTA ACC ATT CAT GAG ATC GAG AAC AAT ACA GAC GAA CTG AAG TTT AGC AAC   3332
Glu Gly Cys Val Thr Ile His Glu Ile Glu Asn Asn Thr Asp Glu Leu Lys Phe Ser Asn
```

EP 0 186 379 B1

# Fig. I-8

```
                                              1070                                                    1080
TGT GTA GAA GAG GAA GTA TAT CCA AAC AAC ACG GTA ACG TGT AAT GAT TAT ACT GCG ACT   3392
Cys Val Glu Glu Glu Val Tyr Pro Asn Asn Thr Val Thr Cys Asn Asp Tyr Thr Ala Thr

                                              1090                                                    1100
CAA GAA GAA TAT GAG GGT ACG TAC ACT TCT CGT AAT CGA GGA TAT GAC GGA GCC TAT GAA   3452
Gln Glu Glu Tyr Glu Gly Thr Tyr Thr Ser Arg Asn Arg Gly Tyr Asp Gly Ala Tyr Glu

                                              1110                                                    1120
AGC AAT TCT TCT GTA CCA GCT GAT TAT GCA TCA GCC TAT GAA GAA AAA GCA TAT ACA GAT   3512
Ser Asn Ser Ser Val Pro Ala Asp Tyr Ala Ser Ala Tyr Glu Glu Lys Ala Tyr Thr Asp

                                              1130                                                    1140
GGA CGA AGA GAC AAT CCT TGT GAA TCT AAC AGA GGA TAT GGG GAT TAC ACA CCA CTA CCA   3572
Gly Arg Arg Asp Asn Pro Cys Glu Ser Asn Arg Gly Tyr Gly Asp Tyr Thr Pro Leu Pro

                                              1150                                                    1160
GCT GGC TAT GTG ACA AAA GAA TTA GAG TAC TTC CCA GAA ACC GAT AAG GTA TGG ATT GAG   3632
Ala Gly Tyr Val Thr Lys Glu Leu Glu Tyr Phe Pro Glu Thr Asp Lys Val Trp Ile Glu

                                              1170                                                    1180
ATC GGA GAA ACG GAA GGA ACA TTC ATC GTG GAC AGC GTG GAA TTA CTT CTT ATG GAG GAA   3692
Ile Gly Glu Thr Glu Gly Thr Phe Ile Val Asp Ser Val Glu Leu Leu Leu Met Glu Glu


TAATATATGCTTTAAAATGTAAGGTGTGCAAATAAAGAATGATTACTGACTTGTATTGACAGATAA   3758


ATAAGGAAATTTTTATATGAATAAAAAACGGGCATCACTCTTAAAAGAATGATGTCCGTTTTTTGT   3824
```

# Fig. I- 9

ATGATTTAACGAGTGATATTTAAATGTTTTTTTGCGAAGGCTTTACTTAACGGGGTACCGCCACAT 3890

GCCCATCAACTTAAGGATAGAAACTCTACGGTTTTCCATTAATTGAAATGAACAAACTTTTAAACA 3956

GTATAATCCTCTTAAAAATGAGCATACGAAATAAACCCTAACGGGTTTCATTTTTTTCTTATGCAG 4022

CTTTCTTTTGTTTTCTCAATCCATTATACTCATAGACAACACCCATAATATCAAAGATAGTTTTCT 4088

TCTCATATCGATGGGATTTCCGGCCATTTTTCTTTAGTAGGGTAAACAGGCGGATTAAAACCTTTG 4154

TTATTATACGGGTGTTTCTCTGTATCGCTTGATATAACAGGGATAGATGATCTTGAATCATCCCAA 4220

TTGCTTTATATTCGCTTAGTTCTCTTTTGTGCTTTTGCAACAGAAGTTGGCGCATCTTAAACATCG 4286

TGGAAGAACATATAAAAATGGCAATGAGTTTTCCATACACATGGCATTCTAATCGCTCTTGTTTGA 4352

TAGTTTGCCAATGATGAATTTGAAATAGAGATTTCCACGTTTTAAATATGATTTCGATCTGCCAGC 4418

GGAGGGAGTAAAAATCATGGATTTGTTCCATCGGAACCACTTCCCAAGGCGTATTCGTAACATATA 4484

TGTTGATACCCGTTAATCGTTTACTCTTTTCTGAAAATGTAATCCCCTTTTTACTTTCGGTATAGC 4550

EP 0 186 379 B1

## Fig. 1-10

```
TTTGTTTTTTTCTACGTTCCAGTATTTGTTCCTCTGTTAATCGGTAGATAATTACTCTTGTAAATA 4616

ATCTTTGATTCTTGCCAATATACGACTCTTTTATTTCATACGTTTGTCCTGGTTTTAACGTGTGCA 4682

TAATGTTCTCTAAATCAACTTGGATATACTGTGACTGTTTTTTGATTGTCCCATTTCGAAAGTATT 4748

CTGGAAATGGATTTTTCGTATAGACGGTATGGTTTAACTTAAGTCTTGATATATAGCACACGCCAC 4814

GTTGATCCATTTGATCTAAGTCTTCCAATGAAAAATATCCTAAATCACGAATACACAGATCACCCG 4880

GCCGTAAGGTATCTAAGCAATCTGTTCCAAATGTTTTATCATTATTTTTTCCAGGACCTACCTGGA 4946

AGTTTAAAAACTGTCCGCTGTGTAAGTCATATTCTAATTGAATCTTGATACCTGCA           5002
```

# Fig. 2

Fig. 3

# Fig. 4

Fig. 5

# Fig. 6

UPPER SYMBOL

B: <u>Bam</u>HI

C: <u>Cla</u>I

E: <u>Eco</u>RI

H: <u>Hind</u> III

K: <u>Kpn</u>I

P: <u>Pvu</u> II

LOWER. SYMBOL

H: <u>Hpa</u>II

S: <u>Sau</u> 3AI

T: <u>Taq</u>I

EP 0 186 379 B1

EP 0 186 379 B1

# Fig. 7

(1) pLBT 291

(2) pKB 5

(3) pKA 2

(4) pMR 4

(5) pHP 1

(6) pSP 801

(7) pSX 8

(8) pBR 322

76K

97K

144K